(19) 

Europäisches
Patentamt
European
Patent Office
Office européen
des brevets

(11) **EP 4 450 970 A1**

(12) **EUROPEAN PATENT APPLICATION**

(43) Date of publication:
**23.10.2024 Bulletin 2024/43**

(21) Application number: **23382366.5**

(22) Date of filing: **19.04.2023**

(51) International Patent Classification (IPC):
*G01N 33/50* (2006.01)     *A61B 5/11* (2006.01)
*A61B 5/00* (2006.01)     *A61B 5/024* (2006.01)
*A61B 5/0205* (2006.01)

(52) Cooperative Patent Classification (CPC):
**A61B 5/1118; A61B 5/0205; A61B 5/02438;
A61B 5/681; A61B 5/7267; G01N 33/68;
G01N 2800/7042**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Startquake S.L.
33161 Asturias (ES)**

(72) Inventor: **The designation of the inventor has not
yet been filed**

(74) Representative: **Longland, Emma Louise et al
Venner Shipley LLP
406 Science Park
Milton Road
Cambridge CB4 0WW (GB)**

(54) **AGING CLOCK MARKERS**

(57) The present invention relates to methods of assessing aging of the human body. It provides methods of determining a biological age of a subject, comprising obtaining first, second, and third activity variables of the subject and/or analyzing a blood sample from the subject to determine the protein expression levels of at least one biomarker selected from the group consisting of PON1, C8B, SERPIND1, APOA2, A2M, APCS, and APOA1; and determining the biological age using the first, second, and third activity variables and/or the protein expression levels, and the chronological age of the subject. The invention also provides kits and devices for use in the methods.

EP 4 450 970 A1

## Description

### Field of the Invention

The present invention relates to methods of assessing aging of the human body. The invention also concerns kits and devices for use in assessing the aging.

### Background of the Invention

Any action of skeletal muscles that leads to energy expenditure and body movement results in physical activity [1]. In the field of sports medicine, it is well known that regular exercise improves healthspan [1-5], especially among older people, reducing the incidence of cardiovascular disease, cancer, diabetes and obesity [6-8]. Moreover, athletes live significantly longer than overall population [9], while moderate-to-high occupational activity contributes to longevity in men [10]. Nevertheless, although fitness seems to extend lifespan by decreasing all-cause mortality risk [11-13], a direct causality remains unproven [14].

Despite all the evidence, a knowledge gap arises when it comes to finding a robust health-/lifespan predictor that considers exercise-related variables. Notably, molecular tools like epigenetic clocks and telomere length have been introduced to quantify longevity in biological terms. Both are considered robust estimators of age, lifespan and mortality risk [15], but epigenetic clocks achieve greater predictive power [16]. The most used DNA methylation-based estimators are: the pantissue epigenetic clock developed by Horvath [17], the age clock designed by Hannum et al. [18], the mortality risk estimator developed by Zhang et al. [19], the PhenoAge lifespan predictor [20], and GrimAge, a DNAm-based clock which strongly predicts mortality risk, lifespan and morbidity [21]. If we define age acceleration (AgeAccel) as the divergence between epigenetic and chronological age, GrimAgeAccel stands out among other epigenetic clocks for its predictive ability for time-to-death, time-to-coronary heart disease, time-to-cancer and aging-decline phenotypes [21,22].

However, epigenetic clocks remain difficult to introduce into routine clinical practice because they involve advanced molecular studies. For this reason, public health policies should rely on widespread tools that can recap lifestyle factors in a practical way, such as wearable technology. In fact, this type of technology most widely-used tool to register daily rest and exercise [23], and its parameters could act as potential biomarkers of health status and lifespan for personalized healthcare [24]. Consequently, a wearable-based predictor that could reproduce epigenetic estimations should be a promising tool to improve health monitoring in a real-time manner. In parallel, discovering reliable blood biomarkers that can quantify biological age is needed to achieve a complete view of the effect of fitness on healthy aging. Although liquid biopsies provide reliable information about a patients' health status [25], little is known about the relationship between physical activity and aging biomarkers.

### Summary of the Invention

Provided herein are methods, devices, and kits for determining the biological age of a subject.

According to a first aspect of the invention, there is provided a method of determining a biological age of a subject, the method comprising: obtaining first, second, and third activity variables of the subject; and determining the biological age using the first, second, and third activity variables and the chronological age of the subject.

A second, related, aspect of the invention provides a method of determining the biological age of a subject, the method comprising analyzing a blood sample from the subject to determine the protein expression levels of at least one biomarker selected from the group consisting of PON1, C8B, SERPIND1, APOA2, A2M, APCS, and APOA1, and determining the biological age of the subject using the expression levels of the at least one biomarker and the chronological age of the subject.

A third aspect of the invention provides a method of determining the biological age of a subject comprising obtaining first, second, and third activity variables of the subject, analyzing a blood sample from the subject to determine the protein expression levels of at least one biomarker selected from the group consisting of PON1, C8B, SERPIND1, APOA2, A2M, APCS, and APOA1, and deytermining the biological age using the first, second, and third activity variables, the protein expression levels of the least one biomarker, and the chronological age of the subject. The third aspect of the invention may be considered a combination of the first and second aspects of the invention.

The subject may be a human. Methods of the above aspects of the invention may be computer implemented. Obtaining activity variables may comprise receiving, or calculating the activity variable.

The first, second, and third activity variables may be or may comprise: minutes of no activity; daily respiratory rate; and resting heart rate.

The resting heart rate may be measured in beats per minute. The daily respiratory rate may be measured using breaths per minute. The minutes of no activity, light activity and high activity may be measured using any suitable means, for example, they may be measured using a wearable device where an activity level has a threshold of motion and/or heart rate, and above or below that threshold, no activity, light activity or hight activity is recorded. The data which may reach a threshold may be recorded from a heart rate monitor, an accelerometer, or a gyroscope. A subject's stress score may be calculated using any suitable means, for example, if using an appropriate wearable device, the score may be measured using electrodermal activity (EDA), measured by electrical pulses in the subject's sweat, or a subject's heart rate. A subject's sleep score may be based on a subject's heart rate, the time the subject spends awake or restless, and the subject's sleep stages.

The activity variables may be measured by any suitable means, and may comprise or consist of descriptive statistics of those variables over a time period, for example maximum, minimum, mean, media, mode, standard deviation and standard error over a time period of minutes, hours, days, weeks, months or years. Preferably the timer period is at least 5 days.

The minutes of no activity may be the number of minutes of no, or not detectable, activity over a time period. The minutes of no activity may be a mean, a weighted mean, or a standard deviation (SD) of the number of minutes over the time period. The daily respiratory rate may be the number of breaths per minute. The daily respiratory rate may be a mean, a weighted mean, or a standard deviation (SD) of the number of breaths per minute over the time period.

One or more of the activity variables may be measured and/or calculated using sensors on a wearable device, for example a smart phone, a smart watch, a Fitbit device, an Apple Watch, or a Garmin watch. The sensors may include an accelerometer, a gyroscope, a heart rate monitor, an optical blood oxygen levels (e.g., a pulse oximeter), a sensor to measure electrical pulses in the subject's sweat and the like.

The minutes of no activity may be the sum of the minutes of no activity for a time period. The daily respiratory rate may be the maximum daily respiratory rate for a time period. The resting heart rate may be a mean resting heart rate for a time period. The time period may be, for example, a day, a week, a fortnight, a month, or a year. The time periods may be the same for all three activity variables.

The first, second and third activity variables may be weighted using a weight determined by a statistical technique, for example a regression, such as a lasso regression. Determining the biological age using the first, second, and third variables may also include applying a constant. The constant may be determined by the statistical technique used to determine the variables, for example, a regression, or a lasso regression.

The biological age may be determined by fitting the first, second and third activity variables and the chronological age to a model.

The model may comprise an independently calculated biological age. The independently calculated biological age may be calculated using the DNAmGrimAge biological clock.

Determining the biological age may comprise using regression. For example, the regression may be a multiple regression. The regression may be linear or non-linear. The lasso regression may comprise a lasso regularisation model.

The first, second and third variables may be obtained from a wearable device. The wearable device may be, for example, a smart watch, a smart bracelet, a smart collar.

Methods may comprise determining the protein expression levels of at least two, at least three, at least four, at least five, at least six, or all seven biomarkers selected from the group consisting of PON1, C8B, SERPIND1, APOA2, A2M, APCS, and APOA1.

Determining the biological age of the subject using the expression levels and the chronological age may comprise fitting the proteins expression levels and the chronological age to a model. Determining the biological age of the subject using the activity variables, expression levels, and the chronological age, may comprise fitting the activity variables, protein expression levels, and the chronological age, to a model. The fitting may be carried out using regression.

The biomarkers, for which expression levels are determined in the method, may consist of one or more biomarkers selected from the group consisting of PON1, C8B, SERPIND1, APOA2, A2M, APCS, and APOA1. Thus PON1, C8B, SERPIND1, APOA2, A2M, APCS, and/or APOA1 may be the only biomarkers for which the protein expression levels are determined in the method.

The biomarkers, for which expression levels are determined, preferably comprise PON1 and/or C8B. The protein expression levels may be determined for a combination of four biomarkers according to a combination listed in Table 8.

Determining the biological age may comprise performing a regression analysis.

Also provided are kits and assay devices that are useful for methods of the invention. Thus, provided is a kit comprising binding partners capable of binding to target molecules representative of protein expression of at least one biomarker selected from the group consisting of PON1, C8B, SERPIND1, APOA2, A2M, APCS, and APOA1. The kit may comprise binding partners capable of binding to target molecules representative of protein expression of at least three, at least four, at least five, at least six, or all seven biomarkers selected from the group consisting of PON1, C8B, SERPIND1, APOA2, A2M, APCS, and APOA1.

Also provided is an assay device comprising a loading area for receipt of a blood sample, binding partners specific for target molecules representative of expression of at least one biomarker selected from the group consisting of PON1, C8B, SERPIND1, APOA2, A2M, APCS, and APOA1, and detection means to detect the levels of said target molecules present in the sample.

Preferably, the target molecules of the assays and kits are the biomarker proteins. The binding partners of the assays and kits may be specific for target molecules representative of protein expression of SERPIND1, C8B, and PON1 and optionally APOA2 and/or APOA1.

According to a fourth aspect of the invention, there is provided a method of determining a biological age of a subject, the method comprising: obtaining at least one activity or health variable of the subject; and determining the biological age using the activity variable and the chronological age of the subject.

The at least one activity or health variable may be one selected from the group of: minutes of no activity; daily respiratory rate; resting heart rate; stress score; sleep score; daily heart rate variability; $VO_2$ max; daily calories burnt; daily step count; blood pressure; smoking history; alcohol consumption history; minutes of light activity; minutes of medium activity; and minutes of high activity.

Preferably the at least one activity variable would be minutes of no activity.

A statistical technique such as a regression analysis, for example, support vector machine (SVMs) algorithms, random forest algorithms, nearest neighbour algorithms, multivariate multiple regression, multiple linear regression, non-linear regressions, generalized linear models, lasso regressions and lasso regularisation models may be used to identify the most relevant activity variables.

The analysis and calculation may be computer implemented.

The resting heart rate may be measured in beats per minute. The daily respiratory rate may be measured using breaths per minute. The minutes of no activity, light activity and high activity may be measured using any suitable means, for example, they may be measured using a wearable device where an activity level has a threshold of motion and/or heart rate, and above or below that threshold, no activity, light activity or hight activity is recorded. The data which may reach a threshold may be recorded from a heart rate monitor, an accelerometer, or a gyroscope. A subject's stress score may be calculated using any suitable means, for example, if using an appropriate wearable device, the score may be measured using electrodermal activity (EDA), measured by electrical pulses in the subject's sweat, or a subject's heart rate. A subject's sleep score may be based on a subject's heart rate, the time the subject spends awake or restless, and the subject's sleep stages.

The activity variables may be measured by any suitable means, and may comprise or consist of descriptive statistics of those variables over a time period, for example maximum, minimum, mean, media, mode, standard deviation and standard error over a time period of minutes, hours, days, weeks, months or years. Preferably the timer period is at least 5 days.

The minutes of no activity may be the number of minutes of no, or not detectable activity over a time period. The minutes of no activity may be a mean, a weighted mean, or a standard deviation (SD) of the number of minutes over the time period. The daily respiratory rate may be the number of breaths per minute. The daily respiratory rate may be a mean, a weighted mean, or a standard deviation (SD) of the number of breaths per minute over the time period.

The variables may be measured and/or calculated using sensors on a wearable device, for example a smart phone, a smart watch, for example, for example, a Fitbit device, an Apple Watch, or a Garmin watch. The sensors may include an accelerometer, a gyroscope, a heart rate monitor, an optical blood oxygen levels (e.g., a pulse oximeter), a sensor to measure electrical pulses in the subject's sweat and the like.

The minutes of no activity may be the sum of the minutes of no activity for a time period. The daily respiratory rate may be the maximum daily respiratory rate for a time period. The resting heart rate may be a mean resting heart rate for a time period. The time period may be, for example, a day, a week, a fortnight, a month, or a year. The time periods may be the same for all three activity variables.

The activity variable may be weighted using a weight determined by a statistical technique, for example a regression, or a lasso regression. Determining the biological age using the activity variable may also include applying a constant. The constant may be determined by the statistical technique used to determine the variables, for example, a regression, or a lasso regression.

The at least one activity variable may be obtained from a wearable device. The wearable device may be, for example, a smart watch, a smart bracelet, a smart collar.

**Brief Description of the Figures**

Figure 1 shows that exercise faithfully predicts GrimAge epigenetic clock. (a) Scatter plot showing the correlation between ActivAge clock and GrimAge using training data (n = 48). (b) Scatter plot depicting the GrimAge predictions of ActivAge clock in test subjects (n = 11). (c) Barplot describing the contribution of each variable to ActivAge model predictions.

Figure 2 shows that the ProAge clock accurately estimates GrimAge values. (a) Scatter plot showing the correlation between ProAge clock predictions and GrimAge epigenetic clock using training data (n = 51). (b) Scatter plot depicting the GrimAge predictions of ProAge clock in test subjects (n = 12). (c) Barplot describing the importance of each feature to ProAge model prediction.

Figure 3 shows ActivAge exercise features and ProAge biomarkers undergo correlated alterations. This graph shows correlations found between the 3 physical activity variables used and proteins used in the ProAge clock.

Figure 4 shows a scatter plot depicting the high linear correlation between ActivAge and ProAge clocks. This graph shows Pearson's correlation found between both of the biological clocks (ActivAge and ProAge).

## Detailed Description

The methods of the present invention provide simple tests and measurements that may be useful in determining the biological age, lifespan, pace of biological aging and/or age acceleration/aging of a subject. The kits and devices of the invention are useful for conducting the methods of the invention.

The invention is based on the inventors' significant and surprising finding that certain physiological variables, such as can be determined by a smartwatch, and the levels of certain protein biomarkers, can be used to calculate the biological age and aging of a subject. The biological age calculated according to the methods of the invention is consistent with the biological age calculated using the widely accepted and validated GrimAge biological clock. Thus, the physiological variables and/or biomarker expression levels can be used to calculate biological age, lifespan and/or age acceleration, and provide important information regarding mortality risk and morbidity.

The inventors are therefore able to provide methods for calculating lifespan and life expectancy that are much easier, cheaper and faster to carry out than the current, gold standard, GrimAge biological clock, but that provide results consistent with GrimAge. The ability to quickly and easily calculate biological age, lifespan and mortality risk in this way means that subjects and their clinicians are able to make informed choices and identify tailored treatment options that can and should be taken by the subject to improve their longevity.

Of course, in some embodiments the methods of the invention may be used in combination, with other methods of the invention and/or with other methods of assessing the health, life expectancy and/or mortality risk of the subject, in which case the combination may advantageously increase sensitivity and understanding compared to use of the other methods on their own, and allow the prioritization of the identification, follow-up and treatment of those most likely to benefit from medical treatment and/or lifestyle changes. Also, the methods of the invention, whether in combination with other methods or not, allow the investigation and monitoring of health status, for example the effects of interventions, intended to lengthen lifespan and/or reduce rate of aging, and/or lifestyle factors or habits may be analysed by carrying out methods of the invention on the subjects, for example before and after the intervention(s) begin or are carried out.

In order to assist the understanding of the present invention, certain terms used herein will now be further defined, and more generally further details of the invention will be given, in the following paragraphs.

*Chronological Age, Biological Age and Lifespan*

The chronological age of a subject is the amount of time from when they were born to when the method is carried out, i.e. it is the number of years since the subject was born. It may also be referred to as the clinical age. Preferably the chronological age is measured as a whole number in years, with the number rounded down if there has been six months or less since the last anniversary of their birthday, and rounded up if there has been more than six months since the last anniversary of their birthday. For example, a subject who is 37 years and 3 months old may have a chronological age of 37, but a subject who is 37 years and 7 months old may have a chronological age of 38. Alternatively, the chronological age may be measured in years and completed months, for example, with the number of months provided as a fraction of a year. For example, a subject who is 37 years and 3 months old may have a chronological age of 37.25 years.

The biological age of a subject is the age reflected by the physiological changes in their body, for example the epigenetic changes. Evidence has shown that individuals with a biological age greater than their chronological age have an increased risk for death, even after accounting for known risk factors. Biological age is measured in the same units as chronological age, and so preferably the biological age is measured in years as a whole number. Alternatively, the biological age may be provided as a fraction number, with the number of months provided as a fraction of a year.

Thus, the biological age provides information regarding how much the subject's body has aged. By comparing the chronological age to the biological age, it is possible to establish the relative health of the subject, for example; a biological age greater than the chronological age suggests the health of the subject is sub-optimal and that their risk of dying is greater than might be expected according to their chronological age, whilst a biological age less that the chronological age suggests the aging of the subject is healthy and that their risk of dying is less than might be expected according to their chronological age.

The biological age may be related to the methylation status of DNA in the subject's cells. The biological age may be, or may be based on, the corresponding biological age that is, would, or could be calculated for the subject using DNAGrimAge. The biological age may be a measure of the pace at which the subject's body has aged.

Age acceleration refers to a comparison between the chronological age and the biological age. A subject having a biological age greater than their chronological age may be considered as experiencing age acceleration, quantified as a positive number. A subject having a biological age less than their chronological age may be considered as experiencing age acceleration quantified as a negative number, which might also be considered age deceleration. A comparison of the biological age and chronological age therefore provides an aging indicator.

A "raw" assessment of age acceleration may be obtained by subtracting the chronological age from the biological age. Residual acceleration, also known as the residual variation, may be obtained from a linear regression of biological age on chronological age.

Aging rate is another concept commonly used to refer to this "divergence" between biological and chronological age and refers to the ratio between biological and chronological age. In this way, a ratio higher than 1 means that biological age is greater than chronological age and thus, that the subject is aging fast. On the other hand, a ratio lower than 1 is directly related to healthy aging. Thus, methods of the invention may comprise determining the biological age, and using the biological age and the chronological age to calculate an age acceleration, for example according to one or more of the above.

The chronological age and biological age can be used to estimate mortality risk and provide an estimate of remaining lifespan. The estimate of remaining lifespan may also be referred to as life expectancy, and refers to the calculation of when the subject is most likely to die of natural causes, and/or how long it will be before the subject will die of natural causes.

The mortality risk of a subject may be understood to be that of the average individual that has a chronological age corresponding to the subject's biological age. For example, a 35 year old subject having a biological age of 40 may be considered to have the mortality risk of an average 40 year old rather than the mortality risk of the average person having a chronological age of 35. Of course, mortality risk by chronological age group is available based on population-wide statistical studies known in the art.

As is explained in detail herein, the methods of the invention have been developed by establishing variables and/or protein biomarkers that can be used to calculate a biological age that aligns with the DNAGrimAge method (also referred to as "GrimAge" or "DNAmGrimAge", herein), as described in Lu, Ake T et al. "DNA methylation GrimAge strongly predicts lifespan and healthspan." Aging vol. 11,2 (2019): 303-327. Therefore, the methods of the invention may provide results, such as biological age, age acceleration, mortality risk and/or expected lifespan, that correspond to those same results calculated for the same subject using GrimAge.

*The subject*

In the context of the methods and medical uses of the present invention, a subject may be any human requiring or desiring the determination of their biological age.

The subject may be undergoing routine examination, for example as part of a health check, and a method in accordance with the invention may be used to assess the general health and mortality risk for the subject. Methods of the invention may be used in addition to, or as an alternative to, other health screens.

The subject may be undergoing routine monitoring of health, fitness and/or mortality risk. They may choose to have their biological age assessed according to methods of the invention in order to assess and/or monitor their own health, fitness and/or mortality risk. Alternatively or in addition, the methods of the invention may be carried out for the subject on the advice of a health care professional.

The subject may present with symptoms or attributes associated with a limited lifespan, for example reduced fitness levels. In such a subject, the methods may be used to indicate whether and to what extent there should be concerns regarding life expectancy, and what areas should be the focus to improve the lifespan.

Alternatively, the subject may be, or appear to be, asymptomatic and/or generally healthy. Suitably an asymptomatic subject may be a subject who is believed to be at elevated risk of reduced life expectancy and elevated mortality risk. Such a subject may be one who has a family history of early death.

The subject may be a subject who will generally benefit from personalized insights as to how their specific lifestyle and habits are affecting their biological age, aging rate, and/or mortality risk. The subject may be asymptomatic and/or not have any particular health concerns, but may want to assess their healthspan and lifespan and/or they may want to assess their rate or aging and mortality risk, perhaps with the aim of improving those characteristics. The subject may be undergoing a therapy, lifestyle plan and/or intervention, and may want to use one or more methods of the invention to assess how successful the therapy, lifestyle plan and/or intervention is in increasing their healthspan and lifespan and/or reducing their rate or aging and mortality risk.

The subject may be an athlete, and may want to monitor or predict a decline in sports performance.

*Activity Variables*

The activity variables are or comprise variables selected from the group: minutes of no activity; daily respiratory rate; resting heart rate; stress score; sleep score; daily heart rate variability; VO$_2$ max; daily calories burnt; daily step count; minutes of light activity; minutes of medium activity; and minutes of high activity.

The resting heart rate may be measured in beats per minute. The daily heart rate variability may be a measure of the entropy of the heart rate or "R-R interval" or "inter-beat interval (IBI) in milliseconds (ms). The daily heartrate variability may be the variance of the heart rate over a day or 24-hour period. The daily respiratory rate may be measured using breaths per minute. VO$_2$ max is the maximum amount of oxygen your body can use. VO$_2$ max may be measured in millilitres of oxygen consumed within 1 minute for a subject divided by the body weight in kilograms of the subject (mL/kg/min). VO$_2$ max may be measured or estimated using any suitable means. For example, where the VO$_2$ max is measured using a wearable device, the VO$_2$ max may be measured by using the subject's resting heart rate and age using the following equation:

$$VO_2 \ max \ = \ k \ \times \ (maximum \ heat \ rate/resting \ heart \ rate)$$

Where k is a constant, maximum heart rate is measured in beats per minute, (for example, the number of beats in 20 seconds, multiplied by 3) and resting heart rate is measured in beats per minute (for example, number of heart beats in 20 seconds multiplied by 3). *k* may equal 15.3.

The daily calories (or joules, or simply "energy") burnt may be measured using any suitable means, for example, if a wearable device is measuring energy burnt, a combination of heart rate, weight, and time performing the exercise may be used to calculate the energy bunt or used by a subject. The daily step count may be the number of steps the subject takes in day. This may be counted by any suitable means, for example, using an accelerometer and/or gyroscope on a wearable device which is configured to detect when a subject has taken a step, or performed a step-like motion. The minutes of no activity, light activity and high activity may be measured using any suitable means, for example, they may be measured using a wearable device where an activity level has a threshold of motion and/or heart rate, and above or below that threshold, no activity, light activity or hight activity is recorded. The data which may reach a threshold may be recorded from a heart rate monitor, an accelerometer, or a gyroscope. A subject's stress score may be calculated using any suitable means, for example, if using an appropriate wearable device, the score may be measured using electrodermal activity (EDA), measured by electrical pulses in the subject's sweat, or a subject's heart rate. A subject's sleep score may be based on a subject's heart rate, the time the subject spends awake or restless, and the subject's sleep stages.

The activity variables may be measured by any suitable means, and may comprise or consist of descriptive statistics of those variables over a time period, for example maximum, minimum, mean, media, mode, standard deviation and standard error over a time period of minutes, hours, days, weeks, months or years. Preferably the time period is at least 5 days, at least 10 days, or at least 15 days.

The minutes of no activity may be the number of minutes of no, or not detectable activity over a time period. The minutes of no activity may be a mean, a weighted mean, or a standard deviation (SD) of the number of minutes over the time period. The daily respiratory rate may be the number of breaths per minute. The daily respiratory rate may be a mean, a weighted mean, or a standard deviation (SD) of the number of breaths per minute over the time period.

The variables may be measured and/or calculated using sensors on a wearable device, for example a smart phone, a smart watch, e.g., a Fitbit device, an Apple Watch, or a Garmin watch, or a wearable collar or necklace. The sensors may include an accelerometer, a gyroscope, a heart rate monitor, an optical blood oxygen levels (e.g., a pulse oximeter), a sensor to measure electrical pulses in the subject's sweat and the like.

The minutes of no activity may be the sum of the minutes of no activity for a time period. The daily respiratory rate may be the maximum daily respiratory rate for a time period. The resting heart rate may be a mean resting heart rate for a time period. The time period may be, for example, a day, a week, a fortnight, a month, or a year. The time periods may be the same for all three activity variables.

As will be explained in more detail later, the first, second and third activity variables may be weighted using a weight determined by a statistical technique, for example a regression, support vector machines (SVMs) algorithms, random forest algorithms, nearest neighbour algorithms, multivariate multiple regression, multiple linear regression, non-linear regressions, generalized linear models, or a lasso regression. Determining the biological age using the first, second, and third variables may also include applying a constant. The constant may be determined by the statistical technique used to determine the variables, for example, a regression, or a lasso regression.

*Other health variables*

There may be other activity or health variables, or biologically relevant information which may be collected and included in a model to determine biological age. For example, blood pressure, may be included, either measured manually and periodically, or measured automatically using a wearable device.

Additional variables may be included in the analysis, for example, health history, e.g., smoking history, alcohol consumption, and other health and medical history data may be included.

*Blood Sample*

The biomarker expression levels are analysed in a biological sample obtained from a subject. The biological sample is a blood sample or a derivative thereof. The blood sample may be a peripheral blood sample, a capillary blood sample, or a venous blood sample. The blood sample may be a whole blood sample.

The blood sample will comprise blood plasma and may be enriched for blood plasma.

The blood sample may be of capillary blood, and may be obtained by fingerprick, also known as fingerstick.

The blood sample may be collected at the subject's convenience, for example at their home or a convenient non-medical or medical location, or the blood sample may be collected at a medical location or business premises. The processing of the blood sample, to determine expression levels of the one or more biomarkers, may then take place at the location of sample collection, or alternatively the blood sample may be provided to an alternative location for processing and completion of a method of the invention.

The sample collection may involve a collection device, such as a dried blood spot card (dbs card), a lateral flow device for home-testing or point of collection testing, or a plasma collection kit with a blood separator.

*Levels of Biomarkers*

Methods of the invention involve looking at the expression levels of biomarkers selected from the list consisting of PON1, C8B, SERPIND1, APOA2, A2M, APCS, and APOA1, i.e. protein biomarkers listed in Table 1 and/or corresponding to the genes listed in Table 1. The methods involve looking at the levels of at least one biomarker in the list, for example one, two, three, four, five, six, or seven of the biomarkers. Preferably the methods involve looking at the expression levels of at least two biomarkers in the list, at least four of the biomarkers in the list, at least six of the biomarkers in the list, or all of the biomarkers in the list. The kits and devices of the invention correspondingly provide binding partners for looking at the levels of one, two, three, four, five, six, or seven of the biomarkers of the invention, in accordance with methods of the invention.

Table 1 Biomarkers for calculating biological age.

| Gene Name | Protein | Contribution to Estimation |
|---|---|---|
| PON1 | Serum paraoxonase/arylesterase 1 | Negative |
| C8B | Human complement protein C8 beta subunit | Positive |
| SERPIND1 | Heparin cofactor 2 | Positive |
| APOA2 | Apolipoprotein A-II | Negative |
| A2M | Alpha-2-macroglobulin | Negative |
| APCS | Serum amyloid P-component | Positive |
| APOA1 | Apolipoprotein A-I | Negative |

Methods of the invention comprise determining the expression levels of at least one protein biomarker expressed from a gene in the list consisting of PON1, C8B, SERPIND1, APOA2, A2M, APCS, and APOA1. Preferably, the at least one protein biomarker will comprise the protein Heparin cofactor 2, expressed from SERPIND1. Preferably, the at least one protein biomarker will comprise C8B.

Where the expression levels of only one or two protein biomarkers are determined in a method of the invention, preferably the protein biomarker(s) will be expressed from SERPIND1 and/or C8B.

Preferably, methods of the invention involving detecting the expression levels of biomarkers will comprise detecting the expression levels of at least four biomarkers, and the biomarkers will comprise a combination of markers according to a combination listed in Table 8. The biomarkers, in methods of the invention comprising determining the expression levels of biomarkers, may consist of a combination of four biomarkers according to a combination listed in Table 8.

The methods of the invention may comprise determining the protein expression levels of SERPIND1, C8B, and PON1 and optionally one or more of APOA2, A2M, APCS, and APOA1. The methods of the invention may comprise determining the expression levels of SERPIND1, C8B, and PON1 and optionally APOA2 and/or APOA1.

It is particularly preferred that the methods of the invention will comprise determining the expression levels of SERPIND1, C8B, APOA2, and PON1. The protein biomarkers for which expression levels are determined in the methods of the invention may consist of SERPIND1, C8B, APOA2, and PON1.

It is particularly preferred that the methods of the invention will comprise determining the expression levels of SERPIND1, C8B, APOA1, and PON1. The protein biomarkers for which expression levels are determined in the methods of the invention may consist of SERPIND1, C8B, APOA1, and PON1.

It will be apparent to the skilled person that the abovementioned combinations of biomarkers represent various minimal marker sets, and additional biomarkers, whether selected from the list of Table 1 or not, may also be included. Alternatively, in some methods, kits and devices of the invention the one, two, three, four, five, six, or seven biomarkers selected from Table 1 may be the only protein biomarkers for which the expression levels are assessed. However, the methods, kits and devices may also provide for the assessment of one or more control target molecules in the biological sample, where the assessment of the control target molecule(s) allow for the accuracy of the assessment mechanism to be tested.

The biological samples are analysed to determine the protein expression levels of the biomarkers. "Gene expression", or more simply "expression" is the process by which information from a gene is used in the synthesis of a functional gene product, such as a protein.

The expression levels of biomarkers according to the invention may be determined by looking at the amount of a target molecule. It is preferred that the target molecule is the protein expressed from the biomarker gene, as listed in Table 1, or a protein fragment thereof. It is also preferred that the protein expression levels are assessed in a whole blood sample or blood plasma sample from the subject.

The levels of the target molecules, which are representative of expression of the biomarkers in the biological sample, may be investigated using specific binding partners. Preferably the levels of the biomarkers in the biological sample are assessed using binding partners specific for the biomarker proteins such as binding partners selected from the group consisting of aptamers and antibodies or antibody fragments.

Suitable classes of binding partners for any given biomarker will be apparent to the skilled person and are discussed further below. The expression levels of the biomarkers in the biological sample may be detected by direct assessment of binding between the protein target molecules and binding partners. The levels of the biomarkers in the biological sample may be detected using a reporter moiety attached to a binding partner. Preferably the reporter moiety is selected from the group consisting of fluorophores; chromogenic substrates; and chromogenic enzymes.

*Binding Partners*

Expression levels of the biomarkers in a biological sample may be investigated using binding partners which bind or hybridize specifically to a target molecule for the biomarkers, or a fragment thereof. Of course, generally it is anticipated that each binding partner will bind or hybridize specifically to just one of the target molecules, for example biomarker proteins, such that the number of binding partners to be used in the method, device or kit will at least equal the number of biomarkers for which the expression levels are to be determined. In relation to the present invention the term 'binding partners' may include any ligands, which are capable of binding specifically to the relevant biomarker and/or peptide variants thereof with high affinity.

Said ligands include but are not limited to proteins, peptides, antibodies, synthetic affinity probes, carbohydrates, lipids, artificial molecules or small organic molecules such as drugs. In certain embodiments the binding partners may be selected from the group consisting of aptamers, antibodies and antibody fragments.

In the context of the present invention, a binding partner specific to a biomarker should be taken as requiring that the binding partner should be capable of binding to at least one target molecule for such biomarker in a manner that can be distinguished from non-specific binding to molecules that are not target molecules for biomarkers. A suitable distinction may, for example, be based on distinguishable differences in the magnitude of such binding.

The binding partner for target molecules that are protein biomarkers may be selected from the group consisting of antibodies, antibody fragments and aptamers.

Commonly, binding partners in the present invention may be isolated or purified. By "purified" is meant that they are substantially free from other cellular components or material, or culture medium.

Many different techniques known in the art are suitable for detecting binding of the target molecule sequence and for high-throughput analysis of protein interactions. Preferably the interactions between the binding partner and biomarker protein will be analysed using antibodies with a fluorescent reporter attached.

The expression level of a particular biomarker may be detected by direct assessment of binding of the target molecule to its binding partner. Suitable examples of such methods in accordance with this embodiment of the invention may utilise techniques such as electro-impedance spectroscopy (EIS) to directly assess binding of binding partners (e.g. antibodies) to target molecules (e.g. biomarker proteins). In certain embodiments of the present invention the binding partner may be an antibody, or antibody fragment, and the detection of the target molecules utilises an immunological method. In certain preferred embodiments of the methods or devices, the immunological method may be an enzyme-linked immunosorbent assay (ELISA) or utilise a lateral flow device.

Methods of the invention comprise detecting expression levels, and therefore may comprise quantification of the amount of the target molecules indicative of expression of the biomarkers that is present in the patient sample. Suitable methods of the invention, in which the amount of the target molecule present has been quantified, and the volume of the patient sample is known, may further comprise determination of the concentration of the target molecules present in the patient sample.

*Reporter Moieties*

In some methods of the present invention the expression levels of biomarker proteins in a biological sample are determined.

In preferred embodiments of the invention, the expression levels of a particular biomarker will be detectable in a biological sample by a high-throughput screening method, for example, relying on detection of an optical signal, for instance using reporter moieties. For this purpose, it may be necessary for the specific binding partner to incorporate a tag, or be labelled with a removable tag, which permits detection of expression. Such a tag may be, for example, a fluorescence reporter molecule translationally-fused to the binding partner, e.g. Green Fluorescent Protein (GFP), Yellow Fluorescent Protein (YFP), Red Fluorescent Protein (RFP), Cyan Fluorescent Protein (CFP) or mCherry. Such a tag may provide a suitable marker for visualisation of biomarker expression since its expression can be simply and directly assayed by fluorescence measurement of the binding partner in vitro.

Alternatively, it may be an enzyme which can be used to generate an optical signal. Tags used for detection of expression may also be antigen peptide tags. Similarly, reporter moieties may be selected from the group consisting of fluorophores; chromogenic substrates; and chromogenic enzymes.

*Calculating Biological Age*

The biological age of a subject may be determined, or calculated, using activity variables, expression levels of biomarkers, or a combination of activity variables and the expression levels of biomarkers. ActiveAge is a calculation of biological age (and an estimate of DNAmGrimAge), using activity variables, ProAge is a calculation of biological age (and an estimate of DNAmGrimAge), using expression levels of biomarkers, and ProActiveAge is a calculation of biological age (and an estimate of DNAmGrimAge), using both activity variables and expression levels of biomarkers. All three calculations use chronological age of a subject. Biological age may be a measure of the pace at which the subject's body has aged.

In the following, illustrative examples will be used regarding the generation of equations for calculating the biological age. Of course, it will be understood that the invention encompasses varying the number of variables (activity variables, biomarker expression levels, or other variables) and the following can be adapted accordingly by incorporating more or fewer of such variables. It will also be understood that a particular model, represented by an equation in the following, including the number of variables, specific weights, and any specific constants, will be specific to the particular protocols used in the method, for example how the variables are recorded and assessed.

The model with which the biological age will be calculated may be generated using statistical methods, such as regression methods, and/or machine learning techniques, and an independently determined biological age of the subjects for whom the variables (activity variables, biomarker expression levels, or other variables) and chronological age have been determined for carrying out a method of the invention. The independently determined biological age may have been determined, for example, using DNAGrimAge (also referred to herein as GrimAge and DNAmGrimAge). Of course, preferably the information from the subject, such as DNA methylation status, for determining the independently determined biological age will be obtained at approximately the same time as the information from the subject for the variables (activity variables, biomarker expression levels, or other variables) according to the method of the invention.

ActiveAge

Statistical analyses may be used to identify the most relevant activity variables, biomarkers, or a combination of activity variable and expression levels of biomarkers, along with their associated weights to apply to the activity variables and biomarkers. The statistical analysis may also be used to identify a constant or other suitable adjustment to apply to the calculation if necessary. The statistical analysis may be any suitable statistical analysis, for example, a regression analysis, SVM algorithms, random forest algorithms, nearest neighbour algorithms, multivariate multiple regression, multiple linear regression, non-linear regressions, generalized linear models, lasso regressions and lasso regularisation models. The analysis and calculation may be computer implemented.

Preferably, the first, second and third variables are minutes of no activity, daily respiratory rate, and resting heart rate. For example, the biological age ("ActiveAge") may be calculated according to Equation 1.

$$ActiveAge = k + w_1 \times MinNoActiv\_SUM + w_2 \times CA + w_3 \times DailyRR\_MAX + w_4 \times RestingHR\_MEAN$$

$$(Equation\ 1)$$

Where $k$ is a constant, $w_1$, $w_2$, $w_3$, and $w_4$ are weights applied to the four predictive variables respectively. *MinNoActiv_SUM* is the sum of minutes of no activity, *CA* is the chronological age, *DailyRR_MAX* is the maximum value of the daily respiratory rate, and *RestingHR_MEAN* is the mean resting heart rate. The constant and the weights may be determined by the lasso regression and regularisation model. For example, $k$ may be 14.2802, $w_1$ may be 4.2659, $w_2$ may be 0.6855, $w_3$ may be 0.5151, and $w_4$ may be 0.4879. ActiveAge may be considered an approximation of DNAmGrimAge.

The present analysis did not include additional health or medical history data, nor did it include other measurable data like blood pressure. However, if such data were to be obtained for individuals within a population, these could be included in the above analysis to determine the variables which contribute most to the variation of biological age. If additional variables such as these are better predictors of biological age (i.e., contributed more to the predictive model), these could be included in the model for determining the biological age of a subject.

While the ActiveAge is preferably determined using the three identified activity variables above, along with the chronological age, the ActiveAge may be calculated using the chronological age and one activity variable, for example, the minutes of no activity.

ProAge

The protein markers comprise one or more biomarkers selected from the group consisting of PON1, C8B, SERPIND1, APOA2, A2M, APCS, and APOA1. For example, the biological age ("ProAge") may be calculated from expression levels of the complete group of seven biomarker proteins and the chronological age (CA), according to Equation 2:

$$ProAge = k - w_1 \times PON1 + w_2 \times C8B + w_3 \times SERPIND1 + w_4 \times CA - w_5 \times APOA2 - w_6 \times A2M + w_7 \times APCS - w_8 \times APOA1$$

$$(Equation\ 2)$$

Where k is a constant, $w_1$, to $w_8$ are weights applied to the eight predictive variables respectively. The constant and the weights may be determined by any suitable statistical method, for example, a regression such as a lasso regression and regularisation model. For example, $k$ may be 17.4986, $w_1$, may be 2.3988, $w_2$ may be 1.4901, $w_3$ may be 0.7343, $w_4$ may be 0.6738, $w_5$ may be 0.6274, $w_6$ may be 0.3564, $w_7$ may be 0.2334, and $w_8$ may be 0.0268. ProAge may be considered an estimate of DNAmGrimAge. PON1, C8B, SERPIND1, APOA2, A2M, APCS, and APOA are the protein expression levels of these biomarkers.

ActiveAge and ProAge (ProActiveAge)

Methods of the invention encompass methods for determining biological age comprising making use of activity variables *and* proteins biomarkers, as well as the chronological age. For example, the biological age ("ProActiveAge") may be calculated or determined from the activity variables, expression levels of the complete group of seven biomarker proteins and the chronological age (*CA*), according to Equation 3:

$$ProActiveAge = k + w_1 \times MinNoActiv\_SUM + w_2 \times CA + w_3 \times DailyRR\_MAX$$
$$+ w_4 \times RestingHR\_MEAN \; w_5 \times PON1 + w_6 \times C8B + w_7 \times SERPIND1$$
$$+ w_8 \times APOA2 - w_9 \times A2M + w_{10} \times APCS - w_{11} \times APOA1$$

$$(Equation \; 3)$$

Where $k$ is a constant, $w_1$, to $w_{11}$, are weights applied to the eleven predictive variables (activity, variables, protein expression variables and chronological age) respectively. *MinNoActiv_SUM* is the sum of minutes of no activity, *CA* is the chronological age, *DailyRR_MAX* is the maximum value of the daily respiratory rate, and *RestingHR_MEAN* is the mean resting heart rate. PON1, C8B, SERPIND1, APOA2, A2M, APCS, and APOA are the expression levels of these biomarkers. The constant and the weights may be determined by any suitable statistical technique, for example, a regression such as a lasso regression and regularisation model.

*Assay Devices and Kits*

Also provided are assay devices and kits for carrying out the methods described above.

Thus, the invention provides an assay device comprising: a) a loading area for receipt of a biological sample; b) binding partners specific for target molecules representative of expression of at least one biomarker selected from the group consisting of PON1, C8B, SERPIND1, APOA2, A2M, APCS, and APOA$_1$; and c) detection means to detect the levels of said target molecules present in the sample.

The detection means suitably comprises means to detect a signal from a reporter moiety, e.g. a reporter moiety as discussed above.

The device is adapted to detect and quantify the levels of said biomarkers present in the biological sample.

The device may be a lateral flow device, for testing and analysis of expression levels. The device may be designed for at-home sample collection and testing of expression levels.

The invention provides kits for use in the above methods, the kits binding partners capable of binding to target molecules representative of expression of at least one biomarker selected from the group consisting of PON1, SERPIND1, APOA2, A2M, APCS, and APOA1. Preferably the kits further comprise indicators capable of indicating when said binding occurs and/or capable of allowing detection of the levels of biomarker protein expression.

Preferably the kits and devices comprise binding partners capable of binding to target molecules representative of expression of two, three, four, five, six, or all seven biomarkers selected from the group consisting of PON1, C8B, SERPIND1, APOA2, A2M, APCS, and APOA1.

As well as the binding partners for the target molecules of the biomarkers, the devices and kits may further comprise binding partners capable of binding to target molecules representative of expression of additional genes. For example, such genes may be "housekeeping genes", which can act as a positive control and/or to normalize expression across samples, and/or such genes may give an indication of the concentration of one or more cell populations within the biological sample.

Preferably said devices and kits provide binding partners capable of binding to target molecules representative of expression of less than 50, 40, 30, 20, 15, or 10 genes, including the biomarkers and any housekeeping or other control genes.

The devices and kits may further comprise instructions for carrying out methods of the invention. It will be understood that the features of the devices and the kits, such as the binding partners, biomarkers, biological sample, etc, may be in accordance with the detailed description provided above for those features.

*Methods of Assessing Mortality Risk, Diagnosis, and Treatment*

The methods, assays and kits provided herein allow an assessment of the aging of a subject. Following the assessment, the subject may be provided with advice or instructions regarding how to improve the results from the aging assessment, for example how to decrease accelerated aging so that a biological age greater than the chronological age can be reduced so that it is closer to the chronological age.

In addition, or alternatively, the methods may comprise informing the subject regarding how the behaviour of the subject could, or is likely to be, influencing the result obtained from the age assessment. For example, the subject could be encouraged to drink more water or to become more active, and following effort to drink more or to become more active they could be informed of the impact this is having, or is likely to be having, on their biological age assessment.

Thus, provided herein are methods of diagnosis comprising diagnosing a subject as having accelerated aging when their biological age is significantly greater than their chronological age.

Also provided herein are methods of treatment comprising determining the biological age of a subject and then treating the subject so as to reduce their biological age calculation. Such treatment may include, for example, prescribing exercise or other means of consistently changing the activity variables obtained in the method, or prescribing diet changes or pharmaceuticals to alter the expression levels of the biomarker proteins.

*Methods of Optimising, Monitoring, and Assessing Health*

The methods, assays and kits provided herein allow an assessment of the biological age, rate of aging, and/or mortality risk of a subject. In the 11th revision of the WHO International Classification of Diseases (ICD-11), WHO have included *"ageing associated decline in intrinsic capacity"* under the $MG_2A$ diagnostic category of symptoms, signs, or clinical findings not elsewhere classified. This reflects the understanding that accelerated, or unhealthy, aging is a condition that may be susceptible to improvement through intervention, and that it is a worthwhile subject of research.

The methods, assays and kits provided herein are therefore useful for investigating aging, the causes of aging, and interventions that will cause rejuvenation and/or aging in a more healthy or decelerated manner. Methods for studying aging and promoting longevity, or promoting a reduction in aging, may comprise methods of the invention and/or make use of assay devices or kits of the invention. For example, the effects of interventions, intended to lengthen lifespan and/or reduce rate of aging, may be analysed by carrying out methods of the invention to determine the biological age of the subject both before and after the intervention(s) begin or are carried out.

Methods of promoting or optimising health in a subject may comprise a method of the invention. Such methods may further comprise therapeutic treatment or implementation of lifestyle changes, with at least one method of the invention carried out both before the treatment or changes, and at least one method of the invention carried out after the treatment or changes, so that the effect on biological age can be determined. Such methods may comprise long term, or ongoing, monitoring of the biological age of the subject, using methods of the invention.

Other aspects and embodiments of the invention provide the aspects and embodiments described above with the term "comprising" replaced by the term "consisting of and the aspects and embodiments described above with the term "comprising" or "consisting" replaced by the term "consisting essentially of.

It is to be understood that the application discloses all combinations of any of the above aspects and embodiments described above with each other, unless the context demands otherwise. Similarly, the application discloses all combinations of the preferred and/or optional features either singly or together with any of the other aspects, unless the context demands otherwise.

Modifications of the above embodiments, further embodiments and modifications thereof will be apparent to the skilled person on reading this disclosure, and as such, these are within the scope of the present invention.

All documents and sequence database entries mentioned in this specification are incorporated herein by reference in their entirety for all purposes.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

**Examples**

Summary of Example

In this study we monitored a wide set of physical activity and functional parameters in 64 subjects over a period of 15 days, using wearable devices and profiling blood proteome with mass spectrometry analyses. Then, through the application of Machine Learning algorithms, we developed two novel biological age clocks called ActivAge and ProAge, which consist of a minimum set of physiological parameters that accurately measure human health-/lifespan using GrimAge as the reference value. We these biological clocks provide more economical, accessible, aging predictors than those currently available.

*What is already known for this topic*

- Exercise contribution to health is paramount to understand how lifestyle factors influence the risk of disease and mortality.

- Further research beyond that currently available in the literature is needed to clarify if physical activity parameters are valid to calculate the levels of biological aging.

*What this study adds*

- ActivAge clock, based on wearable fitness parameters, accurately predicts human lifespan and healthspan reproducing the best known current epigenetic estimations.

- ProAge clock biomarkers estimate healthy aging status showing correlation with different physical activity factors.

- ActivAge and ProAge clocks could be the basis for further research interventions aimed at studying the interplay between sport, aging biomarkers and longevity.

*How this study might affect research, practice, or policy*

- The biological clocks designed in this study do not require cutting-edge epigenetic analyses and therefore, could be implemented in future research that seek to assess the levels of biological aging according to physical activity parameters.

- Sports medicine research should be further combined with molecular and computational studies in order to design algorithms that expand available services for clinical practice.

- Public health policies should rely on wearable technology as an affordable way to personalize healthcare without the need of performing expensive molecular studies.

*Objectives*

The main objectives of this study were: (a) to develop a human healthspan/lifespan predictor that can accurately reproduce epigenetic clocks using physical activity parameters collected by wearable devices; (b) to design a biological aging clock based on peripheral blood biomarkers that can predict epigenetic age; (c) to associate aging blood biomarkers with fitness parameters.

*Methods*

Fifty-nine subjects were daily monitored for resting and exercise habits during a period of 15 days using a Fitbit Charge 4 Smartwatch device. Peripheral whole blood samples were collected from sixty-four individuals (fifty-eight common) to perform DNA methylation analyses and proteome profiling. Lasso penalized regression algorithms were employed to design biological clocks predicting epigenetic GrimAge estimations about healthy aging.

*Results*

We developed two healthspan/lifespan clocks based on exercise parameters and peripheral blood biomarkers called ActivAge and ProAge, respectively. ActivAge was based on 3 wearable-derived parameters (age, minutes of no activity [sum], daily respiratory rate [maximum], resting heart rate [average]), while ProAge consisted of four pro-aging factors (age, C8B, SERPIND1, APCS) and another four anti-aging biomarkers (PON1, APOA2, A2M and APOA1). A high accuracy was obtained for both biological clocks compared to GrimAge gold-standard estimations (ActivAge: r = 0.967, ProAge: r = 0.990). Importantly, blood aging biomarkers, such as PON1, SERPIND1 and APCS, were slightly correlated with exercise-based parameters.

*Conclusions*

ActivAge clock constitutes a real-time accurate predictor of healthy aging based on easy-to-monitor physical activity parameters. ProAge biomarkers are able to reproduce biological age predictions showing associations with exercise lifestyle factors.

*Keywords:* physical activity, fitness, lifestyle, wearable, aging, epigenetic clocks, lifespan, healthspan, longevity, biomarkers

Methods

*Selection of study subjects*

Healthy adult women (n = 36, F) and men (n = 28, M) between 19-96 years were recruited to participate in this study. To ensure an equitable representation across the whole age range, the number of participants was selected to be similar in three intervals of age, preserving sex balance: a) 19-34 years ($n$ = 20, F/M = 10/10), b) 35-60 years ($n$ = 22, F/M = 14/8), and c) 61-96 years ($n$ = 22, F/M = 12/10). Pregnant women were excluded from this study. All the participants came from Asturias, a region of the North of Spain, and thus belong to Hispanic ethnicity. They provided prior informed consent to be part of the study, which was approved by the Clinical Research Ethics Committee of the Principality of Asturias.

*Anthropometric and clinical parameters*

Clinical data from the 64 participants was obtained through questionnaires specifically designed to collect information about chronological age, sex, height, weight, waist diameter, tobacco use (current smoker, former smoker, never smoker), number of family members, allergies and sleep quality, including daily resting time and the consumption of sleeping pills. Some of this information was later contrasted with that collected from wearable devices (e.g., daily sleep).

*Wearable device measurements*

Wearable technology was used to monitor the physiology of the participants according to their physical activity and sleep condition. 59 out of the 64 study subjects wore a Fitbit Charge 4 smartwatch device for 15 days to monitor the following parameters daily: Fitbit's Stress Score, Fitbit's Sleep Score, resting heart rate, daily heart rate variability, daily respiratory rate, $VO_2$ max, daily calories burnt, daily step count, minutes of no activity, minutes of light activity, minutes of medium activity and minutes of high activity. Several statistical values (mean, median, mode, standard deviation, maximum value) were obtained for each of the aforementioned Fitbit parameters to summarize the physiological status of the participants across time.

*Patient and public involvement statement*

Involving public members in our research is critical to ensuring that our findings are meaningful and relevant for society. Therefore we established an advisory group formed by clinicians and bioinformaticians that provided guidance about study design, recruitment strategies, sample collection and dissemination of the potential clinical applications of the study. We also asked for and received feedback from some participants about the questionnaires and their willingness to use the smartwatch in the future.

*Equity, diversity and inclusion statement*

Our study was carefully planned to avoid any bias related to sex across the whole age range. Besides, wearable devices were provided to all the participants avoiding any type of socioeconomical discrimination. Part of the biological samples from older people were collected at home to consider special accessibility needs. Our author team consists of two women and eight men coming from different disciplines (data science, epigenetics...).

*DNA methylation analyses, blood proteome profiling and definition of ActivAge and ProAge clocks using machine learning models*

Detailed information on the molecular and computational aspects of the study, including blood sample collection, DNA methylation analyses, biological age calculation, mass-spectrometry assays for proteome profiling, and penalized regression modelling for ActivAge/ProAge clock design, is provided below.

*Sample collection and DNA methylation analysis*

Blood samples were taken from the 64 subjects during the second week of the study. Genomic DNA was extracted from whole blood cells using the Qiagen Gentra Puregene® Kit and quantified with the Nanodrop-2000C Spectrophotometer. The EZ-96 DNA Methylation Kit conversion protocol (Zymo Research) was used to perform DNA bisulfite conversion. Processed DNA samples were hybridized to Infinium MethylationEPIC BeadChips following the Illumina Infinium HD Methylation Assay protocol.

*Biological age calculation*

MethylationEPIC array data preprocessing was performed using the ChAMP package [M1] within statistical software R. After importing IDAT files, different probes were filtered according to the following criteria: (a) detection p-value of >0.01 in any samples, (b) <3 beads in at least 5% of samples, (c) SNP-related probes (dbSNP v.147), (d) multi-mapping probes [M2], and (e) those located in sex chromosomes. The final number of probes that passed all filters was 734,887.

Epigenetic age was estimated for all the participants in the study using Horvath's Online Age Calculator (https://dnamage.genetics.ucla.edu/) [M3]. The β-values obtained in the previous stage were fed into the Calculator applying Horvath's modified beta-mixture quantile (BMIQ) normalization. Horvath pantissue, Hannum, PhenoAge, and GrimAge values were extracted, and GrimAge clock was selected for downstream analyses.

*Mass-spectrometry assay*

- *Protein digestion for proteomics.* 5μL of plasma of each individual (corresponding to about 300 of protein measured by NanoDrop 1000, Thermo Fisher), were mixed with 5μl of a buffer containing 50mM Tris, 2 % SDS and 100mM DTT, and boiled for 5 minutes for protein denaturation. Proteins were then subjected to filter-aided digestion (Nanosep Centrifugal Devices with Omega Membrane-10K, PALL) according to manufacter's instructions. Cysteine residues were blocked with 50mM iodoacetamide for 1h at room temperature under darkness. Proteins were digested with trypsin (1:30-trypsin:protein, Promega) overnight at 37 °C. After protein digestion, peptides were eluted from the filter, acidified with 25% trifluoroacetic acid (TFA) to a final concentration of 1%, desalted with Oasis cartridges (Waters) following manufacturer's instructions, Speed-vac dried and stored at -20 °C.

- *TMT labelling.* Peptides were subjected to multiplexed isobaric labelling with 10-plex TMT reagents (Thermo Fisher Scientific) following manufacturer's instructions. Briefly, peptide concentration was measured using DirectDetect Infrared Spectrometer (Merck) and 50 μg of peptides from each sample were labelled [M4,M5]. After labelling, peptides were acidified, desalted with Oasis cartridges (Waters), Speed-vac dried and stored at -20 °C. Each TMT experiment contained the samples from 9 individuals and one channel was reserved for reference internal standard constructed by pooling all the samples.

- *LC-MS analysis.* Labelled peptide samples were analyzed using an Ultimate 3000 HPLC system (Thermo Fisher Scientific) coupled via a nanoelectrospray ion source (Thermo Fisher Scientific, Bremen, Germany) to a Q Exactive HF mass spectrometer (Thermo Fisher Scientific). C18-based reverse phase separation was performed using a PepMap 100 C18 5 μm 0.3 × 5 mm as trapping column (Thermo Fisher Scientific) and a PepMap RSLC C18 EASY-Spray column 50 cm × 75 μm ID as analytical column (Thermo Fisher Scientific). Peptides were loaded in buffer A (0.1% formic acid in water (v/v)) and eluted with an acetonitrile gradient consisting of 0-21% buffer B (100% ACN, 0.1% formic acid (v/v)) for 300 min and 21-90% B for 5 min at a flow rate of 200 nl/min. Mass spectra were acquired in data-dependent manner. MS spectra were acquired in the Orbitrap analyser using full ion-scan mode with a mass range of 400-1500 mass-to-charge (m/z) and 70,000 FT resolution. The automatic gain control target was set at $2\times$ 105 with 50 ms maximun injection time. MS/MS was performed using the top-speed acquisition mode with 3s cycle time. HCD fragmentation was performed at 30% of normalized collision energy and MS/MS spectra were analysed at a 60,000 resolution in the Orbitrap.

- *Protein identification and quantification.* For peptide identification MS/MS spectra were searched with the SEQUEST HT algorithm implemented in Proteome Discoverer 2.5 (Thermo Scientific) against a Uniprot database comprised human protein sequences (June 2022, 42673 entries), using trypsin digestion with a maximum of 2 missed cleavages, using Cys carbamidomethylation (57.021464 Da) and TMT labelling at N-terminal end and Lys (229.162932 Da) as fixed modifications, and Met oxidation (15.994915) as dynamic modification. Precursor mass tolerance was set at 800 ppm and fragment mass tolerance at 0.03 Da; precursor charge range was set to 2-4. Results were analyzed using the probability ratio method [M6] and the false discovery rate (FDR) was calculated based on the search of results against the corresponding decoy database using the refined method [M7], with an additional filter for precursor mass tolerance of 15 ppm [M8]. 1% FDR was used as criterion for peptide identification. Protein quantification was performed using models previously developed [M9-M11] with the SanXoT software package [M12]. Quantitative information was extracted from the MS/MS spectra of TMT-labelled peptides. Peptide quantification was analyzed using the WSPP model, which uses raw quantifications as input data and computes the protein $\log_2$-fold changes for each individual with respect to the value of the reference internal standard sample. In this model protein $\log_2$-ratios

are expressed as standardized variables in units of standard deviation according to their estimated variances (Zq values).

*Machine learning analysis using lasso regularization*

Data variables coming from Fitbit wearable measurements and mass-spectrometry profiling were first normalized so that the values were within interval [0,1]. Then, lasso penalized regression models were built using *caret* (v.6.0-93) [M13] and *glmnet* packages (v.4.1-6) [M14] in R statistical software (v.4.0.2). All the plots in this study were designed using the *ggplot$_2$* package (v.3.3.2) [M15].

Specifically, lasso regularization algorithms reduce model complexity by performing a shrinkage of regression coefficients towards zero, which in last term acts as a concomitant feature subset selection by removing the less contributing variables from model estimations [M16]. The lambda parameter ($\lambda$) controls the effect of the penalty term on model coefficients. Lasso regression is ideal for those systems with a low number of high important predictors, while the remaining ones present discrete contributions [M16]. Therefore, biological age clocks like GrimAge are suitable for this type of machine learning model, since chronological age itself always constitutes the main predictor.

The 59 subjects with wearable device data were split in two different sets (training [80%, *n* = 48] / test [20%, *n* = 11]) using pseudo-random number generation (seed: 64). ActivAge clock was defined on the training set using a 10-fold cross-validated lasso regression model with the following parameters: $\alpha$ = 1; $\lambda$ = 0.7055 (seed: 64). Cross-validation is an element resampling method in which the training set is split in *k* complementary groups of approximately equal size, named *folds*. In this way, different *k* estimations of error are computed for each iteration by removing one validation subset in each round to only consider the remaining (*k* - 1) *folds*. A final model is generated by averaging prediction performance over iterations, reducing potential problems like overfitting or structural bias [M4].

Lambda was tuned to minimize the root-mean-square error (RMSE, see below) starting from a grid within interval [-3,3], while alpha was set to 1. Model performance, including Pearson correlation (*r*), root-mean-square error (RMSE, see below) and mean absolute error (MAE, see below), was evaluated using the algorithm outcome values obtained from the *predict* function for both the training and the test sets.

$$RMSE = \sum_{i=1}^{N} |\widehat{y}_i - y_i| \sqrt{\sum_{i=1}^{N} (\widehat{y}_i - y_i)^2 \Big/ N}$$

$$MAE = \frac{1}{n} \sum_{i=1}^{n} |x_i - x|$$

Similarly, after removing one sample with a defective proteome profiling, we defined the ProAge clock using the mass spectrometry data from the 63 remaining participants. First, these subjects were split in two sets, training (80%, *n* = 51) and test (20%, *n* = 12), using pseudo-random number generation (seed: 64). A 10-fold cross-validated lasso model was selected to build the ProAge clock in the training set using the following parameters: $\alpha$ = 1; $\lambda$ = 0.7055 (seed: 64). Lambda was optimized to minimize RMSE within interval [-3,3] by setting alpha to 1. Pearson correlation, RMSE and MAE parameters were calculated using the algorithm outcome values obtained from the *predict* function for both the training and the test sets.

Subsequently, we sought to determine the importance of each variable in computing ActivAge and ProAge values. Predictors were normalized to be within interval [0,1], including chronological age, and ActivAge/ProAge lasso regression models were again estimated following the aforementioned methods. This strategy allowed us to prevent the bias on model coefficients caused by the different dynamic range between the Fitbit/Protein factors [0-1] and the chronological age [19-96 years]. Lastly, variable contribution was estimated using the *varImp* function from the *caret* package.

Results

All the participants in the study were strictly selected to avoid any design bias related to sex or age (Table 2). Anthropometric parameters, which are associated with higher risk of cardiometabolic disease, were similar across our three age ranges. Smoking and sleeping habits, along with Fitbit variable metrics (Table 3), were typical of a healthy, non-sedentary population.

Table 2. Summary of clinical information from study sample.

| Age group | Young (19 - 34 yrs) | | | Middle (35 - 60 yrs) | | | Older (61 - 96 yrs) | | |
|---|---|---|---|---|---|---|---|---|---|
| Sex | F | M | Total | F | M | Total | F | M | Total |
| Number of subjects, *n* | 10 | 10 | 20 | 14 | 8 | 22 | 12 | 10 | 22 |
| Age / yrs, mean (SD) | 26.0 (4.6) | 27.0 (3.5) | 26.7 (4.0) | 51.9 (5.0) | 46.8 (8.3) | 48.3 (7.4) | 77.7 (13.3) | 72.5 (9.5) | 76.1 (11.8) |
| Weight / kg, mean (SD) | 57.1 (8.5) | 79.6 (8.8) | 68.9 (14.2) | 59.1 (7.0) | 81.4 (14.9) | 67.9 (15.3) | 62.8 (14.6) | 75.8 (5.8) | 69.5 (12.8) |
| Height / cm, mean (SD) | 163.7 (7.0) | 173.1 (30.4) | 168.5 (22.0) | 161.4 (6.9) | 181.6 (11.0) | 168.9 (13.1) | 161.5 (4.9) | 172.9 (5.7) | 167.6 (7.8) |
| Current smoker, *n* (%) | 3 (30.0) | 0 (0.0) | 3 (15.0) | 2 (14.3) | 1 (12.5) | 3 (13.6) | 1 (8.3) | 0 (0.0) | 1 (4.5) |
| Former smoker, *n* (%) | 0 (0.0) | 2 (20.0) | 2 (10.0) | 7 (50.0) | 1 (12.5) | 8 (36.4) | 2 (16.7) | 2 (20.0) | 4 (18.2) |
| Never smoker, *n* (%) | 7 (70.0) | 8 (80.0) | 15 (75.0) | 5 (35.7) | 6 (75.0) | 11 (50.0) | 9 (33.3) | 8 (30.0) | 17 (77.3) |
| > 8 hours of sleep per day, *n* (%) | 0 (0.0) | 3 (30.0) | 3 (15.0) | 0 (0.0) | 0 (0.0) | 0 (0.0) | 4 (33.3) | 3 (30.0) | 7 (31.8) |
| 6-8 hours of sleep per day, *n* (%) | 8 (80.0) | 7 (70.0) | 15 (75.0) | 14 (100) | 8 (100) | 22 (100) | 8 (66.7) | 6 (60.0) | 14 (63.6) |
| 4-6 hours of sleep per day, *n* (%) | 2 (20.0) | 0 (0.0) | 2 (10.0) | 0 (0.0) | 0 (0.0) | 0 (0.0) | 1 (10.0) | 0 (0.0) | 1 (4.5) |
| Sleep medication, *n* (%) | 0 (0.0) | 0 (0.0) | 0 (0.0) | 0 (0.0) | 1 (12.5) | 1 (4.5) | 1 (10.0) | 4 (33.3) | 5 (22.7) |

Table 3. Fitbit variable measurements by age and sex distribution. The mean values indicate the mean of the average Fitbit values during 15 days for each group.

| Age group | Young (19 - 34 yrs) | | | Middle (35 - 60 yrs) | | | Older (61 - 96 yrs) | | |
|---|---|---|---|---|---|---|---|---|---|
| Sex | F | M | Total | F | M | Total | F | M | Total |
| Number of subjects, *n* | 8 | 10 | 18 | 11 | 8 | 19 | 12 | 10 | 22 |
| Stress Score, mean (SD) | 77.6 (6.1) | 77.8 (5-3) | 77.7 (5.5) | 78.7 (4.6) | 80.1 (2.8) | 79.3 (3.9) | 73.9 (8.0) | 74.7 (6.5) | 74.2 (7.2) |
| Sleep Score, mean (SD) | 76.5 (4.2) | 75.7 (2.2) | 76.1 (3.1) | 76.9 (3.0) | 74.1 (2.0) | 75.7 (2.9) | 73.4 (10.2) | 72.4 (5-4) | 73.0 (8.2) |
| Resting heart rate / beats per minute, mean (SD) | 63.5 (5.7) | 58.2 (5.3) | 60.5 (6.0) | 64.1 (5-3) | 56.1 (5.4) | 60.6 (6.6) | 66.8 (8.5) | 61.5 (4-5) | 64.3 (7.4) |
| Daily heart rate variability / entropy, mean (SD) | 2.8 (0.3) | 2.9 (0.3) | 2.9 (0.3) | 2.6 (0.3) | 2.7 (0.4) | 2.6 (0.4) | 2.2 (0.4) | 2.1(0.4) | 2.2 (0.4) |
| Daily respiratory rate / breath per minute, mean (SD) | 15.4 (1.4) | 14.9 (1.7) | 15.1 (1.5) | 14.1 (2.0) | 15.4 (1.3) | 14.6 (1.8) | 14.5 (1.7) | 15.9 (2.2) | 15.1 (2.0) |
| VO2 max / mL/kg/min, mean (SD) | 45.9 (11.0) | 51.4 (3.1) | 48.9 (7.8) | 42.8 (5.6) | 49.0 (7.2) | 45.3 (6.9) | 45.1 (5-4) | 50.6 (4.1) | 47.5 (5.5) |
| Daily calories burnt / cal, mean (SD) | 2133.7 (392.7) | 3193.7 (339.0) | 2669.6 (641.2) | 2212.7 (583-1) | 3261.0 (280.5) | 2605.2 (691.6) | 1912.9 (234.1) | 2477.3 (667.0) | 2151.4 (566.2) |
| Daily step count, mean (SD) | 9405.1 (3924.1) | 11392.7 (4001.2) | 10462.2 (3995.8) | 12575.7 (2832.8) | 13038.7 (3576.5) | 12768.6 (3082.4) | 3486.5 (5854.0) | 6217.1 (7392.1) | 4534.9 (6736.1) |
| Minutes of no activity / min, mean (SD) | 662.0 (94.2) | 629.7 (124.8) | 643.9 (109-9) | 606.0 (130.4) | 624.4 (173-4) | 613.7 (146.1) | 660.4 (248.3) | 746.6 (212.5) | 698.3 (229.1) |
| Minutes of light activity / min, mean (SD) | 255.1 (78.9) | 238.1 (102.4) | 245.5 (90.2) | 335.6 (89.2) | 304.1 (98.2) | 321.9 (91.6) | 127.5 (111.7) | 172.2 (108.0) | 146.2 (109.4) |
| Minutes of medium activity / min, mean (SD) | 17.7 (18.2) | 29.9 (17.7) | 23.7 (18.5) | 18.3 (8.4) | 33.9 (12.4) | 23.7 (12.6) | 21.0 (12.9) | 22.1 (20.3) | 21.6 (16.9) |
| Minutes of high activity / min, mean (SD) | 14.3 (17.0) | 37.5 (15.4) | 24.4 (18.6) | 14.6 (14.5) | 37.1 (27.9) | 21.6 (23.4) | 30.9 (17.0) | 43.4 (39.4) | 38.0 (31.4) |

*Physical activity estimates human lifespan and healthspan through ActivAge clock*

To calculate GrimAge for each participant of our study, we profiled the DNA methylation landscape of whole-blood samples using Illumina Infinium MethylationEPIC BeadChip arrays. We then sought to find out if different Fitbit measurements of physical exercise across 15 days could effectively estimate GrimAge values, the best current predictor for human lifespan and healthspan. To this end, we employed machine learning approaches based on lasso penalized regression in 48 training subjects over a wide age range (19-96 years), which allowed us to perform both feature selection and model tuning at the same time. The resulting 10-fold cross-validated lasso regression model was called ActivAge.

The ActivAge clock was based on 4 main predictors related to exercise: (1) the sum of minutes of no activity (*MinNoActiv_SUM*), (2) the chronological age (CA), (3) the maximum value of the daily respiratory rate (*DailyRR_MAX*), and (4) the average resting heart rate (*RestingHR_MEAN*). The optimal model generated from the training data is indicated in Equation 4. All the selected Fitbit features positively contributed to the calculation of GrimAge values.

$$DNAmGrimAge \sim 14.2802 + 4.2659 \times MinNoActiv\_SUM + 0.6855 \times CA +$$
$$0.5151 \times DailyRR\_MAX + 0.4879 \times RestingHR\_MEAN$$

$$(Equation\ 4)$$

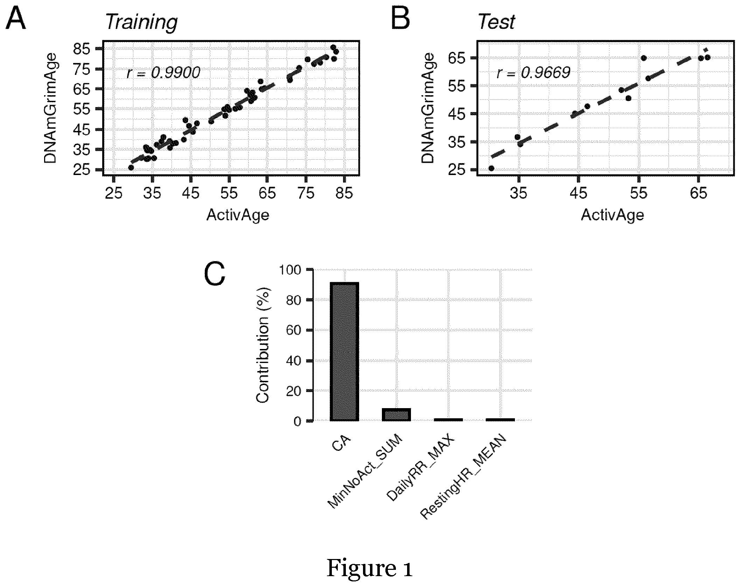

When we evaluated ActivAge performance to model GrimAge, we reached a high Pearson correlation coefficient ($r = 0.9900$) using training individuals (Figure 1A). Other metrics to assess model performance were also adequate (RMSE = 2.5202, MAE = 2.0870). Consequently, we investigated the out-of-sample error of our model, that is to say, how ActivAge clock predicts GrimAge values in subjects that it has not been trained on (Figure 1B). Strikingly, we obtained a high linear correlation using test individuals ($n = 11$, $r = 0.9669$, RMSE = 3.4075, MAE = 2.3618), which constitutes robust evidence of the reliability of our model based on parameters of physical activity.

Next, we proceeded to analyse the contribution of each Fitbit selected feature to the ActivAge model (Figure 1C). As expected, the chronological age was the most relevant variable for obtaining the predicted values (90.92%), followed by the sum of minutes of no activity (7.35%), and the daily respiratory rate (0.89%) and the average resting heart rate (0.84%), which had a similar contribution. These findings underscore the critical importance of the lack of physical activity in the negative determination of lifespan and healthspan beyond chronological age.

*Exercise can influence blood protein biomarkers related to human healthspan and lifespan*

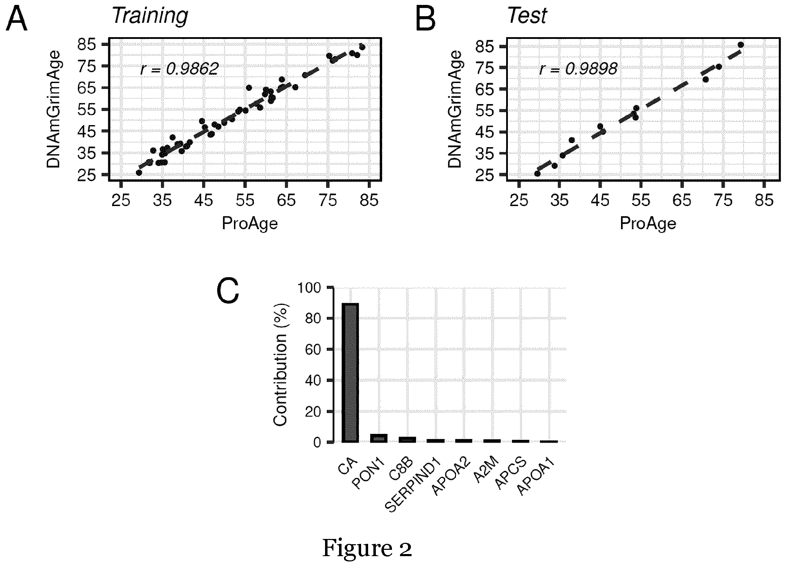

After defining the ActivAge exercise features that predict human lifespan and healthspan, we sought to characterize the protein biomarkers in peripheral blood samples which could better estimate GrimAge clock using the same machine learning approaches. Out of 117 studied proteins, 7 biomarkers (PON1, C8B, SERPIND1, APOA2, A2M, APCS, and APOA1) along with chronological age (CA) could optimally predict GrimAge values in 51 subjects (Figure 2A), with the following metrics: $r = 0.9862$, RMSE = 2.7332, MAE = 2.1330. The resulting model was called ProAge, and its coefficients are indicated in Equation 5. Four proteins (PON1, APOA2, A2M, APOA1) had a negative contribution to ProAge estimations while the remaining half variables (C8B, SERPIND1, CA, APCS) present positive contributions.

$$DNAGrimAge \sim 17.4986 - 2.3988 \times PON1 + 1.4901 \times C8B + 0.7343 \times SERPIND1 +$$
$$0.6738 \times CA - 0.6274 \times APOA2 - 0.3564 \times A2M + 0.2334 \times APCS - 0.0268 \times APOA1$$

$$(Equation\ 5)$$

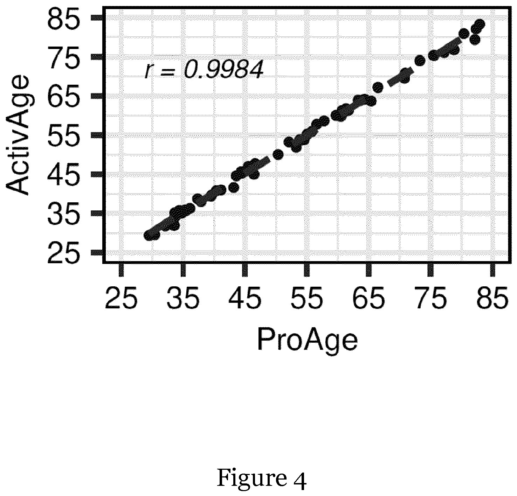

When we analysed the ProAge model performance using 12 test subjects (Figure 2B), we obtained great results, comparable to those of the training set: $r = 0.9898$, RMSE = 3.0660, MAE = 2.5435. In addition, ProAge and ActivAge predictions showed a high correlation, demonstrating the reliability of both models ($r = 0.9984$, Figure 4). Therefore, these collection of peripheral blood biomarkers can effectively predict the GrimAge epigenetic clock without the need of a direct, comprehensive analysis of the DNA methylation.

In Figure 2C we describe the relative contribution of each selected feature to ProAge modelling. Chronological age explained the 89.84% of the prediction, while protein biomarkers performed the remaining 10.16%. Two biomarkers, PON1 and C8B, were the most relevant to calculate predicted values (4.15%, 2.58%, respectively), followed by SERPIND1 and APOA2 contributions (1.27%, 1.09%, respectively). These four proteins, along with CA, represented more than 98% of the model prediction and thus they are the most optimal for the design of a rapid blood test capable of tracking the evolution of individual healthy aging.

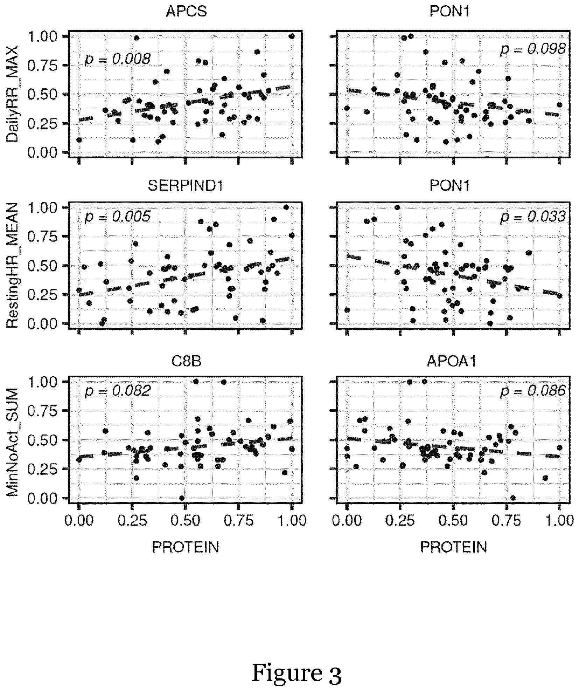

Once we defined the ProAge clock, we sought to answer if the selected ActivAge predictors could be correlated with the levels of the aforementioned biomarkers (Figure 3). First, we observed a clear association between the maximum value of the daily respiratory rate across 15 days (*Daily RR_MAX*) and the blood levels of APCS protein ($p < 0.01$). The PON1 biomarker seemed to show a negative, quasi significant correlation with *DailyRR_MAX* variable ($p = 0.098$). In the case of the average resting heart rate (*RestingHR_MEAN*), we found two proteins whose levels were significantly associated: SERPIND1 ($p < 0.01$) and PON1 ($p < 0.05$). Finally, the sum of minutes of no activity (*MinNoActiv_SUM*), which constitutes the main Fitbit variable to model GrimAge, presented slight associations with the levels of two protein biomarkers, C8B ($p = 0.082$) and APOA1 ($p = 0.086$). Thereby, most of these biomarkers modified their blood levels while the parameters of physical activity were altered.

Discussion

Engaging in regular exercise has been shown to be beneficial in reducing the risk of appearance of non-communicable diseases and increasing overall longevity [26]. In this study, we have implemented machine learning approaches to build reliable fitness-based and protein-based biological aging clocks, called ActivAge and ProAge, using wearable device data and proteome profiling, respectively. To this end, we selected lasso regression algorithms that allowed us to perform both feature selection and model tuning in a single step. With this strategy, we obtained the minimum set of biomarkers that better predict epigenetic GrimAge calculations, removing and penalising those parameters with little or no contribution to modelling.

The ActivAge clock is based on chronological age and three fitness parameters collected by wearable device technology: the sum of minutes of no activity, the maximum value of the daily respiratory rate, and the average resting heart rate. All of these factors are relevant to clarify the mechanisms through which exercise improves human health status. Regarding the main contribution of the minutes of no activity to the algorithm calculation, there is a body of evidence about how sedentary behaviour increases the appearance of adverse health outcomes [27,28]. In fact, moderate-to-high intensity exercise helps to improve overall cardiovascular health status [29]. Similarly, lower resting heart/respiratory rates are directly associated with a better cardiorespiratory fitness that provides cardiovascular protection [30,31]. Therefore, ActivAge represents a promising real-time monitoring tool to personalise health interventions aimed at increasing lifespan and healthspan condition, without the need of performing cutting-edge molecular analyses.

Other studies have attempted to quantify healthy aging based on physical activity parameters [32-35], but they differ in two fundamental aspects. First, our research assessed physical activity during 15 days doubling their time of data collection to better recap the individuals' sport and lifestyle routines. Secondly, the majority of studies considered chronological age as the reference value to be estimated, while the ActivAge clock predicted real biological age through epigenetic predictions, which are more associated with longevity and comorbidities [36].

Likewise, the ProAge clock developed in this study depends on the blood levels of seven biomarkers: PON1, C8B, SERPIND1, APOA2, A2M, APCS and APOA1. These proteins are strongly associated with human inflammation and cardiometabolic status. In fact, PON1 is known for enhancing vascular protection through lipid oxidation [37], while APOA2 levels reduce the risk of future coronary artery disease [38]. Both are high density lipoproteins and constitute the main anti-aging factors of ProAge. Strikingly, our integrative analysis showed that PON1 blood levels are inversely associated with the daily respiratory/resting heart rates, suggesting a worse cardiovascular health. On the contrary, biomarkers such as C8B and SERPIND1 positively contribute to ProAge estimations, in line with their immunity roles. Since inflammation is one of the hallmarks of aging [39], both biomarkers present higher levels in participants with a more sedentary behaviour. Nevertheless, further research will be needed to determine if these systemic biomarkers could constitute therapeutic targets that mimic the positive effects of exercise on human health. Altogether, ProAge represents a promising tool to monitor healthspan and longevity by performing rapid blood tests that would be easy to introduce into routine clinical practice.

*Research and clinical implications*

In this study, we have developed two easy-to-use algorithms, called ActivAge and ProAge, which incorporate exercise features and blood biomarkers with the aim of predicting epigenetic age and healthy aging. ActivAge clock stands on wearable device data expanding the current applications of this technology for personalized healthcare. At the same time, the accuracy of ActivAge predictor allows to rethink public health strategies, based on expensive precision medicine approaches, to develop new technological advances that consider lifestyle factors in a real-time manner. We expect that ActivAge clock could also constitute the basis for future research studies in the field of sports medicine that attempt to quantify aging from a biological perspective.

Regarding the ProAge clock, the selected blood biomarkers are relevant to understand the systemic effects of aging, but beyond these proteins, their associations with lifestyle factors related to sedentary behaviour present broad implications. First, further research will be needed to determine if there is a causal relationship between the intensity of physical activity and the blood levels of ProAge biomarkers. Second, ProAge clock could be a promising personalized tool to assess the benefits of different types of sport interventions on health and longevity.

Our innovative clocks are accessible tools for clinical practice that rely on digitalization and personalization, the pillars for the future medicine. Quantifying the contribution of fitness within precision medicine should be affordable for all socioeconomical status without depending on expensive molecular analyses. To fulfil this purpose, holistic approaches that combine sport, molecular-omics and computational analyses, such as the present study, should guide future studies aimed at enhancing healthcare possibilities.

Conclusions

Our study provides new clinical research tools to quantify biological aging without the need for additional molecular studies. ActivAge clock incorporates real-time wearable measurements associated with sedentary behaviour to accurately predict human lifespan and healthspan. ProAge clock identifies blood biomarkers to estimate healthy aging using simple, rapid blood tests. The association between ProAge clock biomarkers and exercise patterns constitute a way to assess physical activity benefits on systemic healthy aging.

Additional Data

*1.- ProAge VS 20 randomly obtained panels*

Table 4 shows that the selected clock **ProAge,** has the best Pearson's correlation (R = 0.9898) with *GrimAge* (the gold standard) of all panels. MAE (Mean Squared Error) and RMSE (Root Mean Squared Error) are also good and lower than most of them.

Table 4: Comparison between the developed **ProAge** (8-variable panel: 7 proteins plus the chronological age) against 20 "randomly obtained panels" with the same number of proteins.

| Panel | MAE | RMSE | R |
|---|---|---|---|
| **ProAge** | 2.544 | 3.066 | 0.9898 |
| Aleat_1 | 2.599 | 3.007 | 0.9854 |
| Aleat_2 | 2.668 | 3.048 | 0.9850 |
| Aleat_3 | 2.576 | 3.083 | 0.9846 |
| Aleat_4 | 2.730 | 3.239 | 0.9830 |
| Aleat_5 | 2.870 | 3.231 | 0.9831 |
| Aleat_6 | 2.787 | 3.267 | 0.9827 |
| Aleat_7 | 2.871 | 3.236 | 0.9831 |
| Aleat_8 | 3.073 | 4.206 | 0.9712 |
| Aleat_9 | 2.662 | 2.971 | 0.9857 |
| Aleat_10 | 2.445 | 2.868 | 0.9867 |
| Aleat_11 | 2.336 | 2.986 | 0.9856 |
| Aleat_12 | 2.400 | 2.811 | 0.9872 |
| Aleat_13 | 2.659 | 3.254 | 0.9829 |
| Aleat_14 | 2.659 | 3.254 | 0.9829 |
| Aleat_15 | 2.661 | 3.270 | 0.9827 |
| Aleat_16 | 2.722 | 2.943 | 0.9860 |
| Aleat_17 | 2.521 | 3.239 | 0.9830 |
| Aleat_18 | 2.560 | 2.856 | 0.9868 |
| Aleat_19 | 2.730 | 3.320 | 0.9822 |
| Aleat_20 | 2.230 | 2.731 | 0.9880 |

Table 5 shows the individual Pearson's correlation between *GrimAge* and each of the variables that make up *ProAge*. The correlations are higher or lower and positive or negative depending on the protein. Chronological age is the variable that is most correlated.

Table 5: the variables that compose ProAge (ranked by Pearson with GrimAge).

| Variables that compose *ProAge* (ranked by Pearson with *GrimAge*): | |
|---|---|
| 0.98319 | Chronological Age (CA) |
| 0.18471 | Serum amyloid P-component GN=APCS |
| 0.15614 | Heparin cofactor 2 GN=SERPIND1 |
| 0.14112 | Complement component 8 subunit beta GN=C8B |
| -0.09105 | Alpha-2-macroglobulin GN=A2M |
| -0.28882 | Apolipoprotein A-I GN=APOA1 |
| -0.35107 | Apolipoprotein A-II GN=APOA2 |
| -0.45577 | Serum paraoxonase/arylesterase 1 GN=PON1 |

Table 6: Variables that compose the "randomly obtained panels". Note: 'CA' is short for Chronological Age, the only variable that remains constant in all panels. All other variables are provided by protein biomarker name.

| | |
|---|---|
| Aleat_1 | ['CA', LUM, ATIII-R2, APOC3, SNC73, PGLYRP2, HBB, APOE] |
| Aleat_2 | ['CA', IGHM, ITIH3, C1R, APOL1, A2M, LBP, C8B] |
| Aleat_3 | ['CA', HRG, ITIH2, HRG, PLG, AFM, IGL1, FGG] |
| Aleat_4 | ['CA', APCS, CP, F13B, APOE, IGL@, FGG, IGFALS] |
| Aleat_5 | ['CA', IgG L, ATRN, GPLD1, IGHA2, C4BPB, LRG1, CP] |
| Aleat_6 | ['CA', FGB, $C_9$, LPA, KNG1, C4B, TLN1, CPN2] |
| Aleat_7 | ['CA', ORM2, hCG_40889, FN1, IGH@, PGLYRP2, FLNA, IGFALS] |
| Aleat_8 | ['CA', ITIH3, FGA, ITIH1, ATRN, ORM2, SERPINF2, IGK] |
| Aleat_9 | ['CA', SERPINA3, C4BPA, C8G, ITIH4, TTR, SERPINA7, AMBP] |
| Aleat_10 | ['CA', CP, ORM2, AFM, AZGP1, RBP4, FBLN1, FGA] |
| Aleat_11 | ['CA', SERPINA4, C7, RBP4, FGB, C8A, C7, SERPINF2] |
| Aleat_12 | ['CA', CP, C1R, JCHAIN, AZGP1, HPX, IGHG1, HPR] |
| Aleat_13 | ['CA', SERPINA8(1-10), C4B, IgG L, C8G, CP, AZGP1, APOB] |
| Aleat_14 | ['CA', ELSPBP1, APCS, SERPINA4, SERPINA8(1-10), APOA2, SERPINA4, A2M] |
| Aleat_15 | ['CA', ELSPBP1, $ITIH_1$, HBB, Igk, SERPINA8(1-10), transferrin, FGB] |
| Aleat_16 | ['CA', FGB, HP, VTN, APOC3, F13B, AFM, CD5L] |
| Aleat_17 | ['CA', AMBP, LRG1, ITIH2, C4A, CP, A1BG, TTR] |
| Aleat_18 | ['CA', C1R, HP, FGG, C8B, VTN, HRG, DKFZp686G11190] |
| Aleat_19 | ['CA', ITIH4, ITIH2, IgG L, SERPINA8(1-10), C8G, PLG, Igk] |
| Aleat_20 | ['CA', A1BG, ATIII-R2, SERPINA4, ELSPBP1, IgG L, hCG_40889, ATIII-R2] |

*2.- All ProAge possible combinations using 4 proteins*

Table 7 provides the statistical analysis for all possible combinations of **ProAge** in groups of 4 proteins.

Table 7: All possible combinations of **ProAge** in groups of 4 proteins.

| Panel | MAE | RMSE | R |
|---|---|---|---|
| *ProAge_4Prot* | 2.188 | 2.665 | 0.9885 |
| Comb_1 | 2.515 | 2.880 | 0.9866 |
| Comb_2 | 2.160 | 2.577 | 0.9893 |
| Comb_3 | 2.514 | 2.952 | 0.9859 |
| Comb_4 | 2.018 | 2.537 | 0.9896 |
| Comb_5 | 2.269 | 2.840 | 0.9870 |
| Comb_6 | 2.540 | 3.121 | 0.9842 |
| Comb_7 | 2.229 | 2.899 | 0.9864 |
| Comb_8 | 2.284 | 2.889 | 0.9865 |
| Comb_9 | 2.085 | 2.717 | 0.9881 |
| Comb_10 | 2.192 | 2.882 | 0.9866 |
| Comb_11 | 2.525 | 3.115 | 0.9843 |
| Comb_12 | 2.143 | 2.698 | 0.9882 |
| Comb_13 | 2.208 | 2.654 | 0.9886 |
| Comb_14 | 2.477 | 3.023 | 0.9852 |
| Comb_15 | 2.568 | 3.247 | 0.9829 |
| Comb_16 | 2.304 | 2.855 | 0.9868 |
| Comb_17 | 2.451 | 2.897 | 0.9864 |
| Comb_18 | 2.342 | 2.722 | 0.9880 |
| Comb_19 | 2.465 | 2.854 | 0.9868 |
| Comb_20 | 2.459 | 2.871 | 0.9867 |
| Comb_21 | 2.730 | 3.161 | 0.9838 |
| Comb_22 | 2.370 | 2.901 | 0.9864 |
| Comb_23 | 2.702 | 3.250 | 0.9829 |
| Comb_24 | 2.467 | 3.077 | 0.9847 |
| Comb_25 | 2.307 | 2.733 | 0.9879 |
| Comb_26 | 2.229 | 2.794 | 0.9874 |
| Comb_27 | 2.201 | 2.656 | 0.9886 |
| Comb_28 | 2.399 | 2.894 | 0.9865 |
| Comb_29 | 2.328 | 2.683 | 0.9884 |
| Comb_30 | 2.009 | 2.488 | 0.9900 |
| Comb_31 | 2.117 | 2.538 | 0.9896 |
| Comb_32 | 2.607 | 2.945 | 0.9860 |
| Comb_33 | 2.209 | 2.782 | 0.9875 |
| Comb_34 | 2.453 | 2.871 | 0.9867 |

The panel named *Comb_30* provides the best metrics (R = 0.9900, MAE = 2.009 and RMSE = 2.488). However, as shown in Table 7, the other 34 possible combinations are also very good (minimal R = 0.9829) and so provide protein sets for providing accurate predictions of lifespan.

*ProAge_4Prot,* discussed above, is made up of the 4 proteins that most contribute to **ProAge** (obtained by evaluating the weights that each of the 7 proteins has in the algorithm).

Table 8: Provides the biomarker combinations used to generate Table 7.

| Potential combinations in groups of 4 variables: | | | | | | | |
|---|---|---|---|---|---|---|---|
| *ProAge_4Prot* | | SERPIND1 | C8B | | | APOA2 | PON1 |
| Comb_1 | APCS | SERPIND1 | C8B | A2M | | | |
| Comb_2 | APCS | SERPIND1 | C8B | | APOA1 | | |
| Comb_3 | APCS | SERPIND1 | C8B | | | APOA2 | |
| Comb_4 | APCS | SERPIND1 | C8B | | | | PON1 |
| Comb_5 | APCS | SERPIND1 | | A2M | APOA1 | | |
| Comb_6 | APCS | SERPIND1 | | A2M | | APOA2 | |
| Comb_7 | APCS | SERPIND1 | | A2M | | | PON1 |
| Comb_8 | APCS | SERPIND1 | | | APOA1 | APOA2 | |
| Comb_9 | APCS | SERPIND1 | | | APOA1 | | PON1 |
| Comb_10 | APCS | SERPIND1 | | | | APOA2 | PON1 |
| Comb_11 | APCS | | | | APOA1 | APOA2 | PON1 |
| Comb_12 | | SERPIND1 | | | APOA1 | APOA2 | PON1 |
| Comb_13 | | | C8B | | APOA1 | APOA2 | PON1 |
| Comb_14 | | | | A2M | APOA1 | APOA2 | PON1 |
| Comb_15 | APCS | | | A2M | | APOA2 | PON1 |
| Comb_16 | | SERPIND1 | | A2M | | APOA2 | PON1 |
| Comb_17 | | | C8B | A2M | | APOA2 | PON1 |
| Comb_18 | | SERPIND1 | C8B | A2M | APOA1 | | |
| Comb_19 | | | C8B | A2M | APOA1 | APOA2 | |
| Comb_20 | APCS | | C8B | A2M | APOA1 | | |
| Comb_21 | APCS | | C8B | A2M | | APOA2 | |
| Comb_22 | APCS | | C8B | A2M | | | PON1 |
| Comb_23 | APCS | | | A2M | APOA1 | APOA2 | |
| Comb_24 | APCS | | | A2M | APOA1 | | PON1 |
| Comb_25 | | | C8B | A2M | APOA1 | | PON1 |
| Comb_26 | | SERPIND1 | | A2M | APOA1 | | PON1 |
| Comb_27 | | SERPIND1 | C8B | A2M | | | PON1 |
| Comb_28 | APCS | | C8B | | | APOA2 | PON1 |
| Comb_29 | | SERPIND1 | C8B | | APOA1 | APOA2 | |
| Comb_30 | | SERPIND1 | C8B | | APOA1 | | PON1 |
| Comb_31 | APCS | | C8B | | APOA1 | | PON1 |
| Comb_32 | | SERPIND1 | C8B | A2M | | APOA2 | |
| Comb_33 | | SERPIND1 | | A2M | APOA1 | APOA2 | |

(continued)

| Potential combinations in groups of 4 variables: | | | | | | |
|---|---|---|---|---|---|---|
| Comb_34 | APCS | | C8B | | APOA1 | APOA2 | |

*3.- ActivAge VS 20 randomly obtained panels*

Table 9 shows that the selected clock *ActivAge*, has the best Pearson's correlation (R = 0.9669) with *GrimAge* of all panels. MAE and RMSE metrics are also good and higher than most of them.

Table 9: Comparison between the developed *ActivAge* (4-variable panel: 3 physical activity variables plus the chronological age) against 20 "randomly obtained panels" with the same number of physical activity variables.

| Panel | MAE | RMSE | R |
|---|---|---|---|
| *ActivAge* | 2.362 | 3.407 | 0.9669 |
| Aleat_1 | 2.087 | 3.351 | 0.9652 |
| Aleat_2 | 2.265 | 3.359 | 0.9651 |
| Aleat_3 | 2.665 | 3.789 | 0.9553 |
| Aleat_4 | 2.431 | 3.403 | 0.9641 |
| Aleat_5 | 2.453 | 3.467 | 0.9627 |
| Aleat_6 | 2.761 | 3.501 | 0.9620 |
| Aleat_7 | 2.233 | 3.313 | 0.9660 |
| Aleat_8 | 2.691 | 4.016 | 0.9497 |
| Aleat_9 | 2.244 | 3.420 | 0.9638 |
| Aleat_10 | 2.340 | 3.453 | 0.9630 |
| Aleat_11 | 2.537 | 3.834 | 0.9542 |
| Aleat_12 | 2.481 | 3.695 | 0.9576 |
| Aleat_13 | 2.643 | 3.987 | 0.9504 |
| Aleat_14 | 2.372 | 3.500 | 0.9620 |
| Aleat_15 | 2.398 | 3.930 | 0.9518 |
| Aleat_16 | 2.479 | 3.609 | 0.9595 |
| Aleat_17 | 2.340 | 3.445 | 0.9632 |
| Aleat_18 | 2.348 | 3.356 | 0.9651 |
| Aleat_19 | 2.390 | 3.501 | 0.9620 |
| Aleat_20 | 2.300 | 3.469 | 0.9627 |

Table 10 shows the correlation for each of the variables that make up *ActivAge.* The specific correlations are vary depending on the variable. As anticipated and consistent with the findings of GrimAge, chronological age is the variable that is most correlated.

Table 10: The individual Pearson's correlation between *GrimAge* and each of the variables that make up *ActivAge.*

| Variables that compose *ActivAge* (Ranked by Pearson with *GrimAge*): | |
|---|---|
| 0.985 | Chronological Age (CA) |
| 0.367 | Daily Respiratory Rate MAX |
| 0.364 | Minutes of No Activity SUM |

(continued)

| Variables that compose *ActivAge* (Ranked by Pearson with *GrimAge*): | |
|---|---|
| 0.265 | Resting Heart Rate MEAN |

Table 11: Variables that compose the "randomly obtained panels" used to generate the data of Table 9 and referenced in Table 9. CA: Chronological Age; Stress Score: Fitbit's Stress Score; Sleep Score: Fitbit's Sleep Score; RestingHR: Resting Heart Rate; DailyHRV: Daily Heart Rate Variability; DailyRR: Daily Respiratory Rate; VO2Max: Amount (volume) of oxygen required by the body while exercising; Calories: Calories burnt; Steps: Steps; MinNoAct: Minutes of No Activity; MinActLig: Minutes of Light Activity; MinActMe: Minutes of Medium Activity; MinActHigh: Minutes of High Activity; MOD: 15-day study mode; MAX: 15-day study maximum value; DESV: 15-day study standard deviation; SUM: 15-day study sum; MEAN: 15-day study average; MED: 15-day study median.

| | |
|---|---|
| Aleat_1 | ['CA', StressScore_MOD, Calories_DESV, MinActHigh_MED] |
| Aleat_2 | ['CA', DailyHRV_DESV, Calories_MAX, Steps_MEAN] |
| Aleat_3 | ['CA', Vo2Max_MED, MinActHigh_DESV, StressScore_MOD] |
| Aleat_4 | ['CA', Calories_DESV, RestingHR_SUM, DailyRR_MEAN] |
| Aleat_5 | ['CA', SleepScore_MED, DailyRR_MEAN, StressScore_ MED] |
| Aleat_6 | ['CA', MinNoAct_MED, RestingHR _ SUM, Vo2Max_MEAN] |
| Aleat_7 | ['CA', DailyHRV_DESV, MinNoAct_ MAX, SleepScore _ MAX] |
| Aleat_8 | ['CA', RestingHR_MEAN, DailyHRV_ MAX, Vo2Max_MED] |
| Aleat_9 | ['CA', DailyHRV_MAX, RestingHR_DESV, DailyRR_DESV] |
| Aleat_10 | ['CA', SleepScore_MOD, MinActLig_DESV, SleepScore_ MEAN] |
| Aleat_11 | ['CA', RestingHR_MAX, Calories_MAX, MinActMe_MEAN] |
| Aleat_12 | ['CA', MinActHigh_DESV, DailyHRV_DESV, MinActMe_MED] |
| Aleat_13 | ['CA', RestingHR_MOD, SleepScore_MEAN, DailyRR_SUM] |
| Aleat_14 | ['CA', MinActHigh_DESV, DailyRR_ MEAN, Vo2Max_MAX] |
| Aleat_15 | ['CA', RestingHR_DESV, MinActHigh_MEAN, RestingHR_ MAX] |
| Aleat_16 | ['CA', MinActHigh_MAX, MinActLig_DESV, Steps_MEAN] |
| Aleat_17 | ['CA', MinActMe_MAX, MinActMe_SUM, MinActLig_MAX] |
| Aleat_18 | ['CA', DailyHRV_MAX, Calories_MAX, MinActLig_SUM] |
| Aleat_19 | ['CA', SleepScore_MAX, MinActHigh_DESV, MinActMe_ SUM] |
| Aleat_20 | ['CA', SleepScore_DESV, MinActMe_ DESV, Vo2Max_ MEAN] |

*4.- ProAge + ActivAge combination*

Table 12: Metrics for the combination of *ProAge* and *ActivAge*.

| Panel | MAE | RMSE | R |
|---|---|---|---|
| ***ProAge + ActivAge*** | 2.646 | 3.294 | 0.9708 |

It is shown in Table 12 that the combination of *ProAge* + *ActivAge* has optimal RMSE and R (R = 0.9708 and RMSE = 3.294) than the *ActivAge* itself (R = 0.9669 and RMSE = 3.407), so this combination improves *ActivAge's* accuracy.

Table 13 shows the individual Pearson's correlation between *GrimAge* and each of the variables that make up *ProAge* and *ActivAge.* The correlations are higher or lower and positive or negative depending on the variable. As anticipated and consistent with the findings of GrimAge, chronological age is the variable that is most correlated.

Table 13: individual Pearson's correlation between *GrimAge* and each of the variables that make up *ProAge* and *ActivAge.*

| Variables that compose *ProAge* + *ActivAge* (Ranked by Pearson with *GrimAge*): | |
|---|---|
| 0.985 | Chronological Age (CA) |
| 0.3891 | Daily Respiratory Rate MAX |
| 0.3646 | Minutes of No Activity SUM |
| 0.2657 | Resting Heart Rate MEAN |
| 0.1726 | Serum amyloid P-component GN=APCS |
| 0.1669 | Heparin cofactor 2 GN=SERPIND1 |
| 0.1309 | Complement component 8 subunit beta GN=C8B |
| -0.0805 | Alpha-2-macroglobulin GN=A2M |
| -0.3048 | Apolipoprotein A-I GN=APOA1 |
| -0.4007 | Apolipoprotein A-II GN=APOA2 |
| -0.4543 | Serum paraoxonase/arylesterase 1 GN=PON1 |

*5.- Statistical methods used for obtaining the panels*

*ProAge* and *ActivAge* were generated using the lasso penalized regression model. For more information, please see section "**Error! Reference source not found.**" in the Methods section, above.

The 35 combinations of *ProAge* in groups of 4 proteins were generated manually. There are exactly 35 combinations for grouping 7 different variables in groups of 4. See below the mathematical equation for obtaining how many combinations exist.

$$(n, r) \ = \ n! \, / \, (r! \ * \ (n \ - \ r)!)$$

$$\text{(Equation 6)}$$

Where n is the number of proteins in the big group (7)

And r is how many proteins we want to include in each combination (4)

*ProAge* + *ActivAge* optimal combination were calculated using linear regression for the group of CA + 7 proteins + 3 physical activity variables.

*6.- Metrics Used for Panels Evaluation*

**R:** Pearson's correlation between the panel prediction and GrimAge prediction. The skilled person will be familiar with such calculations, but see also "Machine learning analysis using lasso regularization" in the Methods section, above.

**MAE:** Mean Squared Error between the panel prediction and GrimAge prediction. See formula below. The skilled person will be familiar with such calculations, but see also "Machine learning analysis using lasso regularization" in the Methods section, above.

$$MAE = \frac{1}{n}\sum_{i=1}^{n}|x_i - x|$$

Equation 7

Where n is the number of samples,

$x_i$ is the panel prediction of the i sample, and

$x$ is the GrimAge prediction of the *i* sample

**RMSE:** Root Mean Squared Error between the panel prediction and GrimAge prediction. See formula below. The skilled person will be familiar with such calculations, but see also "Machine learning analysis using lasso regularization" in the Methods section, above.

$$RMSE = \sqrt{\frac{\sum_{i=1}^{N}(Predicted_i - Actual_i)^2}{N}}$$

Equation 8

- Where $N$ is the number of samples,
- $Predicted_i$: panel prediction of the i sample, and
- $Actual_i$: GrimAge prediction of the i sample

References

All reference cited herein are incorporated by reference in their entirety.

1 Langhammer B, Bergland A, Rydwik E. The Importance of Physical Activity Exercise among Older People. BioMed Research International 2018;2018:e7856823. doi:10.1155/2018/7856823

2 Taylor D. Physical activity is medicine for older adults. Postgraduate Medical Journal 2014;90:26-32. doi:10.1136/postgradmedj-2022-131366

3 Hamer M, Lavoie KL, Bacon SL. Taking up physical activity in later life and healthy ageing: the English longitudinal study of ageing. Br J Sports Med 2014;48:239-43. doi:10.1136/bjsports-2013-092993

4 Gopinath B, Kifley A, Flood VM, et al. Physical Activity as a Determinant of Successful Aging over Ten Years. Sci Rep 2018;8:10522. doi:10.1038/s41598-018-28526-3

5 Moreno-Agostino D, Daskalopoulou C, Wu Y-T, et al. The impact of physical activity on healthy ageing trajectories: evidence from eight cohort studies. International Journal of Behavioral Nutrition and Physical Activity 2020;17:92. doi:10.1186/s12966-020-00995-8

6 Ahmed HM, Blaha MJ, Nasir K, et al. Effects of Physical Activity on Cardiovascular Disease. The American Journal of Cardiology 2012;109:288-95. doi:10.1016/j.amjcard.2011.08.042

7 Garcia L, Pearce M, Abbas A, et al. Non-occupational physical activity and risk of cardiovascular disease, cancer and mortality outcomes: a dose-response meta-analysis of large prospective studies. Br J Sports Med Published Online First: 24 January 2023. doi:10.1136/bjsports-2022-105669

8 Cleven L, Krell-Roesch J, Nigg CR, et al. The association between physical activity with incident obesity, coronary heart disease, diabetes and hypertension in adults: a systematic review of longitudinal studies published after 2012. BMC Public Health 2020;20:726. doi:10.1186/s12889-020-08715-4

9 Runacres A, Mackintosh KA, McNarry MA. Health Consequences of an Elite Sporting Career: Long-Term Detriment or Long-Term Gain? A Meta-Analysis of 165,000 Former Athletes. Sports Med 2021;51:289-301. doi:10-1007/s40279-020-01379-5

10 Dalene KE, Tarp J, Selmer RM, et al. Occupational physical activity and longevity in working men and women in Norway: a prospective cohort study. The Lancet Public Health 2021;6:e386-95. doi:10.1016/S2468-2667(21)00032-3

11 Reimers CD, Knapp G, Reimers AK. Does Physical Activity Increase Life Expectancy? A Review of the Literature. Journal of Aging Research 2012;2012:e243958. doi:10.1155/2012/243958

12 Gorzelitz J, Trabert B, Katki HA, et al. Independent and joint associations of weightlifting and aerobic activity with all-cause, cardiovascular disease and cancer mortality in the Prostate, Lung, Colorectal and Ovarian Cancer Screening Trial. Br J Sports Med 2022;56:1277-83. doi:10.1136/bjsports-2021-105315

13 Coleman CJ, McDonough DJ, Pope ZC, et al. Dose-response association of aerobic and muscle-strengthening physical activity with mortality: a national cohort study of 416 420 US adults. Br J Sports Med 2022;56:1218-23. doi:10-1136/bjsports-2022-105519

14 Kujala UM. Is physical activity a cause of longevity? It is not as straightforward as some would believe. A critical analysis. Br J Sports Med 2018;52:914-8. doi:10.1136jbjsports-2017-098639

15 Marioni RE, Harris SE, Shah S, et al. The epigenetic clock and telomere length are independently associated with chronological age and mortality. International Journal of Epidemiology 2016;45:424-32. doi:10.1093/ije/dyw041

16 Jylhävä J, Pedersen NL, Hägg S. Biological Age Predictors. EBioMedicine 2017;21:29-36. doi:10.1016/j.ebiom.2017.03.046

17 Horvath S. DNA methylation age of human tissues and cell types. Genome Biology 2013;14:3156. doi:10.1186/gb-2013-14-10-r115

18 Hannum G, Guinney J, Zhao L, et al. Genome-wide Methylation Profiles Reveal Quantitative Views of Human Aging Rates. Molecular Cell 2013;49:359-67. doi:10.1016/j.molcel.2012.10.016

19 Zhang Y, Wilson R, Heiss J, et al. DNA methylation signatures in peripheral blood strongly predict all-cause mortality. Nat Commun 2017;8:14617. doi:10.1038/ncomms14617

20 Levine ME, Lu AT, Quach A, et al. An epigenetic biomarker of aging for lifespan and healthspan. Aging 2018;10:573-91. doi:10.18632/aging.101414

21 Lu AT, Quach A, Wilson JG, et al. DNA methylation GrimAge strongly predicts lifespan and healthspan. Aging 2019;11:303-27. doi:10.18632/aging.101684

22 McCrory C, Fiorito G, Hernandez B, et al. GrimAge Outperforms Other Epigenetic Clocks in the Prediction of Age-Related Clinical Phenotypes and All-Cause Mortality. The Journals of Gerontology: Series A 2021;76:741-9. doi:10.1093/gerona/glaa286

23 Tokuçoğlu F. Monitoring Physical Activity with Wearable Technologies. *Archives of Neuropsychiatry* 2018;**55**:S63. doi:10.29399/npa.23333

24 Iqbal SMA, Mahgoub I, Du E, et al. Advances in healthcare wearable devices, npj Flex Electron 2021;5:1-14. doi:10.1038/s41528-021-00107-x

25 Mayeux R. Biomarkers: Potential Uses and Limitations. NeuroRx 2004;1:182-8.

26 Lee I-M, Shiroma EJ, Lobelo F, et al. Effect of physical inactivity on major non-communicable diseases worldwide: an analysis of burden of disease and life expectancy. The Lancet 2012;380:219-29. doi:10.1016/S0140-6736(12)61031-9

27 Cao Z, Xu C, Zhang P, et al. Associations of sedentary time and physical activity with adverse health conditions: Outcome-wide analyses using isotemporal substitution model. eClinicalMedicine 2022;48. doi:10.1016/j.eclinm.2022.101424

28 Jingjie W, Yang L, Jing Y, et al. Sedentary time and its association with risk of cardiovascular diseases in adults: an updated systematic review and meta-analysis of observational studies. BMC Public Health 2022;22:286. doi:10.1186/s12889-022-12728-6

29 Rognmo Ø, Moholdt T, Bakken H, et al. Cardiovascular Risk of High- Versus Moderate-Intensity Aerobic Exercise in Coronary Heart Disease Patients. Circulation 2012;126:1436-40. doi:10.1161/CIRCULATIONAHA.112.123117

30 Al-Mallah MH, Sakr S, Al-Qunaibet A. Cardiorespiratory Fitness and Cardiovascular Disease Prevention: an Update. Curr Atheroscler Rep 2018;20:1. doi:10.1007/s11883-018-0711-4

31 Raghuveer G, Hartz J, Lubans DR, et al. Cardiorespiratory Fitness in Youth: An Important Marker of Health: A Scientific Statement From the American Heart Association. Circulation 2020;142:e101-18. doi:10.1161/CIR.0000000000000866

32 McIntyre RL, Rahman M, Vanapalli SA, et al. Biological Age Prediction From Wearable Device Movement Data Identifies Nutritional and Pharmacological Interventions for Healthy Aging. Frontiers in Aging 2021;2.https://www.frontiersin.org/articles/10-3389/fragi.2021.708680 (accessed 29 Mar 2023).

33 Pyrkov TV, Getmantsev E, Zhurov B, et al. Quantitative characterization of biological age and frailty based on locomotor activity records. Aging 2018;10:2973-90. doi:10.1632/aging.101603

34 Rahman SA, Adjeroh DA. Deep Learning using Convolutional LSTM estimates Biological Age from Physical Activity. Sci Rep 2019;9:11425. doi:10.1038/s41598-019-46850-0

35 Pyrkov TV, Slipensky K, Barg M, et al. Extracting biological age from biomedical data via deep learning: too much of a good thing? Sci Rep 2018;8:5210. doi:10.1038/s41598-018-23534-9

36 Marioni RE, Shah S, McRae AF, et al. DNA methylation age of blood predicts all-cause mortality in later life. Genome Biology 2015;16:25. doi:10.1186/s13059-015-0584-6

37 Furlong CE, Suzuki SM, Stevens RC, et al. Human PON1, a biomarker of risk of disease and exposure. Chemico-Biological Interactions 2010;187:355-61. doi:10.1016/j.cbi.2010.03.033

38 Birjmohun RS, Dallinga-Thie GM, Kuivenhoven JA, et al. Apolipoprotein A-II Is Inversely Associated With Risk of Future Coronary Artery Disease. Circulation 2007;116:2029-35. doi:10.1161/CIRCULATIONAHA.107.704031

39 López-Otín C, Blasco MA, Partridge L, et al. The Hallmarks of Aging. Cell 2013;153:1194-217. doi:10.1016/j.cell.2013.05.039

**SUPLEMENTARY REFERENCES FROM METHODS SECTION**

M1 Morris TJ, Butcher LM, Feber A, et al. ChAMP: 450k Chip Analysis Methylation Pipeline. Bioinformatics 2014;30:428-30. doi:10.1093/bioinformatics/btt684

M2 Zhou W, Laird PW, Shen H. Comprehensive characterization, annotation and innovative use of Infinium DNA methylation BeadChip probes. Nucleic Acids Res 2017;45:e22. doi:10.1093/nar/gkw967

M3 Horvath S. DNA methylation age of human tissues and cell types. Genome Biology 2013;14:3156. doi:10.1186/gb-2013-14-10-r115

M4 Baldan-Martin M, Lopez JA, Corbacho-Alonso N, et al. Potential role of new molecular plasma signatures on cardiovascular risk stratification in asymptomatic individuals. Sci Rep 2018;8:4802.

doi:10.1038/s41598-018-23037-7

M5 Bagwan N, Bonzon-Kulichenko E, Calvo E, et al. Comprehensive Quantification of the Modified Proteome Reveals Oxidative Heart Damage in Mitochondrial Heteroplasmy. Cell Rep 2018;23:3685-3697.e4. doi:10.1016/j.celrep.2018.05.080

M6 Martiánez-Bartolomé S, Navarro P, Martian-Maroto F, et al. Properties of Average Score Distributions of SEQUEST: The Probability Ratio Method. Molecular & Cellular Proteomics 2008;7:1135-45. doi:10.1074/mcp.M700239-MCP200

M7 Navarro P, Vazquez J. A Refined Method To Calculate False Discovery Rates for Peptide Identification Using Decoy Databases. J Proteome Res 2009;8:1792-6. doi:10.1021/pr800362h

M8 Bonzon-Kulichenko E, Garcia-Marques F, Trevisan-Herraz M, et al. Revisiting Peptide Identification by High-Accuracy Mass Spectrometry: Problems Associated with the Use of Narrow Mass Precursor Windows. J Proteome Res 2015;14:700-10. doi:10.1021/pr5007284

M9 Navarro P, Trevisan-Herraz M, Bonzon-Kulichenko E, et al. General Statistical Framework for Quantitative Proteomics by Stable Isotope Labeling. J Proteome Res 2014;13:1234-47. doi:10.1021/pr4006958

M10 Jorge I, Navarro P, Martinez-Acedo P, et al. Statistical Model to Analyze Quantitative Proteomics Data Obtained by 18O/16O Labeling and Linear Ion Trap Mass Spectrometry: Application to the Study of Vascular Endothelial Growth Factor-induced Angiogenesis in Endothelial Cells. Molecular & Cellular Proteomics 2009;8:1130-49. doi:10.1074/mcp.M800260-MCP200

M11 García-Marqués F, Trevisan-Herraz M, Martinez-Martinez S, et al. A Novel Systems-Biology Algorithm for the Analysis of Coordinated Protein Responses Using Quantitative Proteomics. Molecular & Cellular Proteomics 2016;15:1740-60. doi:10.1074/mcp.M115.055905

M12 Trevisan-Herraz M, Bagwan N, García-Marqués F, et al. SanXoT: a modular and versatile package for the quantitative analysis of high-throughput proteomics experiments. Bioinformatics 2019;35:1594-6. doi:10.1093/bioinformatics/bty815

M13 Kuhn M. Building Predictive Models in R Using the caret Package. Journal of Statistical Software 2008;28:1-26. doi:10.18637/jss.v028.i05

M14 Tibshirani R, Bien J, Friedman J, et al. Strong Rules for Discarding Predictors in Lasso-Type Problems. Journal of the Royal Statistical Society Series B: Statistical Methodology 2012;74:245-66. doi:10.1111/j.1467-9868.2011.01004.x

M15 Wickham H. ggplot2: Elegant Graphics for Data Analysis. New York, NY: : Springer 2009. doi:10.1007/978-0-387-98141-3

M16 James G, Witten D, Hastie T, et al. An Introduction to Statistical Learning. New York, NY: : Springer 2013. doi:10.1007/978-1-4614-7138-7

## Claims

1. A method of determining a biological age of a subject, the method comprising:

   obtaining first, second, and third activity variables of the subject; and
   determining the biological age using the first, second, and third activity variables and the chronological age of the subject.

2. The method of claim 1, wherein the first, second, and third activity variables are or comprise:

   minutes of no activity;

daily respiratory rate; and
resting heart rate.

3. The method claim 1 or 2, wherein determining the biological age comprises fitting the first, second and third activity variables and the chronological age to a model.

4. The method of any preceding claim, wherein the first, second and third variables are obtained from a wearable device.

5. A method of determining the biological age of a subject, the method comprising:

analyzing a blood sample from the subject to determine the protein expression levels of at least one biomarker selected from the group consisting of PON1, C8B, SERPIND1, APOA2, A2M, APCS, and APOA1; and determining the biological age of the subject using the expression levels of the at least one biomarker and the chronological age of the subject.

6. A method of determining the biological age of a subject, the method comprising:

obtaining first, second, and third activity variables of the subject;
analyzing a blood sample from the subject to determine the protein expression levels of at least one biomarker selected from the group consisting of PON1, C8B, SERPIND1, APOA2, A2M, APCS, and APOA1; and determining the biological age using the first, second, and third activity variables, the protein expression levels of the at least one biomarker, and the chronological age of the subject.

7. A method according to claim 5 or claim 6, comprising determining the protein expression levels of at least two, at least three, at least four, at least five, at least six, or all seven biomarkers selected from the group consisting of PON1, C8B, SERPIND1, APOA2, A2M, APCS, and APOA1.

8. A method according to any of claims 5 to 7, wherein determining the biological age of the subject using the expression levels and the chronological age comprises fitting the protein expression levels and the chronological age to a model.

9. A method according to any of claims 5 to 8, wherein the biomarkers, for which expression levels are determined in the method, consist of one or more biomarkers selected from the group consisting of PON1, C8B, SERPIND1, APOA2, A2M, APCS, and APOA1.

10. A method according to any of claims 5 to 9, wherein the biomarkers, for which expression levels are determined, comprise PON1 and/or C8B.

11. A method according to any of claims 5 to 10 wherein the protein expression levels are determined of a combination of biomarkers according to a combination listed in Table 8.

12. A method of any preceding claim, wherein determining the biological age comprises performing a regression analysis.

13. A kit comprising binding partners capable of binding to target molecules representative of protein expression of at least one biomarker selected from the group consisting of PON1, C8B, SERPIND1, APOA2, A2M, APCS, and APOA1, preferably at least three, at least four, at least five, at least six, or all seven biomarkers selected from the group consisting of PON1, C8B, SERPIND1, APOA2, A2M, APCS, and APOA1.

14. An assay device comprising:

a loading area for receipt of a blood sample;
binding partners specific for target molecules representative of expression of at least one biomarker selected from the group consisting of PON1, C8B, SERPIND1, APOA2, A2M, APCS, and APOAL; and detection means to detect the levels of said target molecules present in the sample.

15. A kit according to claim 13 or an assay device according to claim 14 wherein:

i) the target molecules are the biomarker proteins; and/or
ii) the binding partners are specific for target molecules representative of protein expression of SERPIND1, C8B, and PON1 and optionally APOA2 and/or APOA1.

Figure 1

Figure 2

Figure 3

Figure 4

## EUROPEAN SEARCH REPORT

Application Number

EP 23 38 2366

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/228237 A1 (SHALLENBERGER FRANK [US]) 13 October 2005 (2005-10-13) * abstract * * paragraphs [0006] – [0046] * ----- | 1-4 | INV. G01N33/50 A61B5/11 A61B5/00 A61B5/024 |
| X | RAHMAN SYED ASHIQUR ET AL: "Estimating Biological Age from Physical Activity using Deep Learning with 3D CNN", 2019 IEEE INTERNATIONAL CONFERENCE ON BIOINFORMATICS AND BIOMEDICINE (BIBM), IEEE, 18 November 2019 (2019-11-18), pages 1100-1103, XP033704082, DOI: 10.1109/BIBM47256.2019.8983251 * Abstract; Introduction; Methodology; Results; Discussion; Conclusion * ----- | 1-4 | A61B5/0205 |
| X | NAKAMURA E ET AL: "Assessment of biological age by principal component analysis", MECHANISMS OF AGEING AND DEVELOPMENT, ELSEVIER SEQUOIA, LAUSANNE, CH, vol. 46, no. 1-3, 1 January 1988 (1988-01-01), pages 1-18, XP023429301, ISSN: 0047-6374, DOI: 10.1016/0047-6374(88)90109-1 [retrieved on 1988-01-01] * Summary; Introduction; Materials and Methods; Results; Discussion * ----- | 1-4 | |
| X | WO 2005/110460 A2 (UNIV OHIO [US]; KOPCHICK JOHN J [US] ET AL.) 24 November 2005 (2005-11-24) | 5-12 | |
| Y | * page 389; claims 3, 4; table 1C * ----- | 6 | |
| | | | TECHNICAL FIELDS SEARCHED (IPC) G01N A61B |

-/--

The present search report has been drawn up for all claims

| Place of search | Date of completion of the search | Examiner |
|---|---|---|
| Munich | 6 December 2023 | Schwachtgen, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KARASIK D. ET AL: "Disentangling the Genetic Determinants of Human Aging: Biological Age as an Alternative to the Use of Survival Measures", JOURNALS OF GERONTOLOGY, SERIES A, BIOLOGICAL SCIENCES ANDMEDICAL SCIENCES, vol. 60, no. 5, 1 May 2005 (2005-05-01), pages 574-587, XP093107683, US ISSN: 1079-5006, DOI: 10.1093/gerona/60.5.574 Retrieved from the Internet: URL:https://academic.oup.com/biomedgeronto logy/article-pdf/60/5/574/1496586/574.pdf> [retrieved on 2023-12-05] | 5,7-12 | |
| Y | * table 3 * | 6 | |

TECHNICAL FIELDS
SEARCHED      (IPC)

The present search report has been drawn up for all claims

| Place of search | Date of completion of the search | Examiner |
|---|---|---|
| Munich | 6 December 2023 | Schwachtgen, J |

EPO FORM 1503 03.82 (P04C01)

page 2 of 2

Europäisches
Patentamt

European
Patent Office

Office européen
des brevets

**Application Number**

**EP 23 38 2366**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-4(completely); 5-12(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

**Application Number**

EP 23 38 2366

The Search Division considers that the present European patent application does not comply with the requirements of unity of invention and relates to several inventions or groups of inventions, namely:

```
1. claims: 1-4

    Methods of determining biological age using physiological
    variables
                        ---

2-3. claims: 5-12(partially)

    Methods of determining biological age of a subject using
    PON1 or C8B, respectively
                        ---

4-8. claims: 5-9, 11, 12(all partially)

    Methods of determining biological age of a subject using
    SERPIND1, APOA2, A2M, APCS, or APOA1, respectively
                        ---

9. claims: 13-15

    Kit and apparatus for binding molecules in the blood samples
                        ---
```

## ANNEX TO THE EUROPEAN SEARCH REPORT
## ON EUROPEAN PATENT APPLICATION NO.

EP 23 38 2366

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2023

| Patent document cited in search report | Publication date | Patent family member(s) | Publication date |
|---|---|---|---|
| US 2005228237 A1 | 13-10-2005 | NONE | |
| WO 2005110460 A2 | 24-11-2005 | NONE | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LU, AKE T et al.** DNA methylation GrimAge strongly predicts lifespan and healthspan. *Aging,* 2019, vol. 11 (2), 303-327
- **LANGHAMMER B ; BERGLAND A ; RYDWIK E.** The Importance of Physical Activity Exercise among Older People. *BioMed Research International,* 2018, vol. 2018, e7856823
- **TAYLOR D.** Physical activity is medicine for older adults. *Postgraduate Medical Journal,* 2014, vol. 90, 26-32
- **HAMER M ; LAVOIE KL ; BACON SL.** Taking up physical activity in later life and healthy ageing: the English longitudinal study of ageing. *Br J Sports Med,* 2014, vol. 48, 239-43
- **GOPINATH B ; KIFLEY A ; FLOOD VM et al.** Physical Activity as a Determinant of Successful Aging over Ten Years. *Sci Rep,* 2018, vol. 8, 10522
- **MORENO-AGOSTINO D ; DASKALOPOULOU C ; WU Y-T et al.** The impact of physical activity on healthy ageing trajectories: evidence from eight cohort studies. *International Journal of Behavioral Nutrition and Physical Activity,* 2020, vol. 17, 92
- **AHMED HM ; BLAHA MJ ; NASIR K et al.** Effects of Physical Activity on Cardiovascular Disease. *The American Journal of Cardiology,* 2012, vol. 109, 288-95
- **GARCIA L ; PEARCE M ; ABBAS A et al.** Non-occupational physical activity and risk of cardiovascular disease, cancer and mortality outcomes: a dose-response meta-analysis of large prospective studies. *Br J Sports Med,* 24 January 2023
- **CLEVEN L ; KRELL-ROESCH J ; NIGG CR et al.** The association between physical activity with incident obesity, coronary heart disease, diabetes and hypertension in adults: a systematic review of longitudinal studies published after 2012. *BMC Public Health,* 2020, vol. 20, 726
- **RUNACRES A ; MACKINTOSH KA ; MCNARRY MA.** Health Consequences of an Elite Sporting Career: Long-Term Detriment or Long-Term Gain? A Meta-Analysis of 165,000 Former Athletes. *Sports Med,* 2021, vol. 51, 289-301
- **DALENE KE ; TARP J ; SELMER RM et al.** Occupational physical activity and longevity in working men and women in Norway: a prospective cohort study. *The Lancet Public Health,* 2021, vol. 6, e386-95
- **REIMERS CD ; KNAPP G ; REIMERS AK.** Does Physical Activity Increase Life Expectancy? A Review of the Literature. *Journal of Aging Research,* 2012, vol. 2012, e243958
- **GORZELITZ J ; TRABERT B ; KATKI HA et al.** Independent and joint associations of weightlifting and aerobic activity with all-cause, cardiovascular disease and cancer mortality in the Prostate, Lung, Colorectal and Ovarian Cancer Screening Trial. *Br J Sports Med,* 2022, vol. 56, 1277-83
- **COLEMAN CJ ; MCDONOUGH DJ ; POPE ZC et al.** Dose-response association of aerobic and muscle-strengthening physical activity with mortality: a national cohort study of 416 420 US adults. *Br J Sports Med,* 2022, vol. 56, 1218-23
- **KUJALA UM.** Is physical activity a cause of longevity? It is not as straightforward as some would believe. A critical analysis. *Br J Sports Med,* 2018, vol. 52, 914-8
- **MARIONI RE ; HARRIS SE ; SHAH S et al.** The epigenetic clock and telomere length are independently associated with chronological age and mortality. *International Journal of Epidemiology,* 2016, vol. 45, 424-32
- **JYLHÄVÄ J ; PEDERSEN NL ; HÄGG S.** Biological Age Predictors. *EBioMedicine,* 2017, vol. 21, 29-36
- **HORVATH S.** DNA methylation age of human tissues and cell types. *Genome Biology,* 2013, vol. 14, 3156
- **HANNUM G ; GUINNEY J ; ZHAO L et al.** Genome-wide Methylation Profiles Reveal Quantitative Views of Human Aging Rates. *Molecular Cell,* 2013, vol. 49, 359-67
- **ZHANG Y ; WILSON R ; HEISS J et al.** DNA methylation signatures in peripheral blood strongly predict all-cause mortality. *Nat Commun,* 2017, vol. 8, 14617
- **LEVINE ME ; LU AT ; QUACH A et al.** An epigenetic biomarker of aging for lifespan and healthspan. *Aging,* 2018, vol. 10, 573-91
- **LU AT ; QUACH A ; WILSON JG et al.** DNA methylation GrimAge strongly predicts lifespan and healthspan. *Aging,* 2019, vol. 11, 303-27
- **MCCRORY C ; FIORITO G ; HERNANDEZ B et al.** GrimAge Outperforms Other Epigenetic Clocks in the Prediction of Age-Related Clinical Phenotypes and All-Cause Mortality. *The Journals of Gerontology: Series A,* 2021, vol. 76, 741-9

- **IQBAL SMA ; MAHGOUB I ; DU E et al.** Advances in healthcare wearable devices. *npj Flex Electron*, 2021, vol. 5, 1-14
- **MAYEUX R.** Biomarkers: Potential Uses and Limitations. *NeuroRx*, 2004, vol. 1, 182-8
- **LEE I-M ; SHIROMA EJ ; LOBELO F et al.** Effect of physical inactivity on major non-communicable diseases worldwide: an analysis of burden of disease and life expectancy. *The Lancet*, 2012, vol. 380, 219-29
- **CAO Z ; XU C ; ZHANG P et al.** Associations of sedentary time and physical activity with adverse health conditions: Outcome-wide analyses using isotemporal substitution model. *eClinicalMedicine*, 2022, vol. 48
- **JINGJIE W ; YANG L ; JING Y et al.** Sedentary time and its association with risk of cardiovascular diseases in adults: an updated systematic review and meta-analysis of observational studies. *BMC Public Health*, 2022, vol. 22, 286
- **ROGNMO Ø ; MOHOLDT T ; BAKKEN H et al.** Cardiovascular Risk of High- Versus Moderate-Intensity Aerobic Exercise in Coronary Heart Disease Patients. *Circulation*, 2012, vol. 126, 1436-40
- **AL-MALLAH MH ; SAKR S ; AL-QUNAIBET A.** Cardiorespiratory Fitness and Cardiovascular Disease Prevention: an Update. *Curr Atheroscler Rep*, 2018, vol. 20, 1
- **RAGHUVEER G ; HARTZ J ; LUBANS DR et al.** Cardiorespiratory Fitness in Youth: An Important Marker of Health: A Scientific Statement From the American Heart Association. *Circulation*, 2020, vol. 142, e101-18
- **MCINTYRE RL ; RAHMAN M ; VANAPALLI SA et al.** Biological Age Prediction From Wearable Device Movement Data Identifies Nutritional and Pharmacological Interventions for Healthy Aging. *Frontiers in Aging*, 29 March 2023, vol. 2, https://www.frontiersin.org/articles/10-3389/fragi.2021.708680
- **PYRKOV TV ; GETMANTSEV E ; ZHUROV B et al.** Quantitative characterization of biological age and frailty based on locomotor activity records. *Aging*, 2018, vol. 10, 2973-90
- **RAHMAN SA ; ADJEROH DA.** Deep Learning using Convolutional LSTM estimates Biological Age from Physical Activity. *Sci Rep*, 2019, vol. 9, 11425
- **PYRKOV TV ; SLIPENSKY K ; BARG M et al.** Extracting biological age from biomedical data via deep learning: too much of a good thing?. *Sci Rep*, 2018, vol. 8, 5210
- **MARIONI RE ; SHAH S ; MCRAE AF et al.** DNA methylation age of blood predicts all-cause mortality in later life. *Genome Biology*, 2015, vol. 16, 25
- **FURLONG CE ; SUZUKI SM ; STEVENS RC et al.** Human PON1, a biomarker of risk of disease and exposure. *Chemico-Biological Interactions*, 2010, vol. 187, 355-61

- **BIRJMOHUN RS ; DALLINGA-THIE GM ; KUIVENHOVEN JA et al.** Apolipoprotein A-II Is Inversely Associated With Risk of Future Coronary Artery Disease. *Circulation*, 2007, vol. 116, 2029-35
- **LÓPEZ-OTÍN C ; BLASCO MA ; PARTRIDGE L et al.** The Hallmarks of Aging. *Cell*, 2013, vol. 153
- **MORRIS TJ ; BUTCHER LM ; FEBER A et al.** ChAMP: 450k Chip Analysis Methylation Pipeline. *Bioinformatics*, 2014, vol. 30, 428-30
- **ZHOU W ; LAIRD PW ; SHEN H.** Comprehensive characterization, annotation and innovative use of Infinium DNA methylation BeadChip probes. *Nucleic Acids Res*, 2017, vol. 45, e22
- **BALDAN-MARTIN M ; LOPEZ JA ; CORBACHO-ALONSO N et al.** Potential role of new molecular plasma signatures on cardiovascular risk stratification in asymptomatic individuals. *Sci Rep*, 2018, vol. 8, 4802
- **BAGWAN N ; BONZON-KULICHENKO E ; CALVO E et al.** Comprehensive Quantification of the Modified Proteome Reveals Oxidative Heart Damage in Mitochondrial Heteroplasmy. *Cell Rep*, 2018, vol. 23, 3685-3697.e4
- **MARTIÁNEZ-BARTOLOMÉ S ; NAVARRO P ; MARTIAN-MAROTO F et al.** Properties of Average Score Distributions of SEQUEST: The Probability Ratio Method. *Molecular & Cellular Proteomics*, 2008, vol. 7, 1135-45
- **NAVARRO P ; VAZQUEZ J.** A Refined Method To Calculate False Discovery Rates for Peptide Identification Using Decoy Databases. *J Proteome Res*, 2009, vol. 8, 1792-6
- **BONZON-KULICHENKO E ; GARCIA-MARQUES F ; TREVISAN-HERRAZ M et al.** Revisiting Peptide Identification by High-Accuracy Mass Spectrometry: Problems Associated with the Use of Narrow Mass Precursor Windows. *J Proteome Res*, 2015, vol. 14, 700-10
- **NAVARRO P ; TREVISAN-HERRAZ M ; BONZON-KULICHENKO E et al.** General Statistical Framework for Quantitative Proteomics by Stable Isotope Labeling. *J Proteome Res*, 2014, vol. 13, 1234-47
- **JORGE I ; NAVARRO P ; MARTINEZ-ACEDO P et al.** Statistical Model to Analyze Quantitative Proteomics Data Obtained by 18O/16O Labeling and Linear Ion Trap Mass Spectrometry: Application to the Study of Vascular Endothelial Growth Factor-induced Angiogenesis in Endothelial Cells. *Molecular & Cellular Proteomics*, 2009, vol. 8, 1130-49
- **GARCÍA-MARQUÉS F ; TREVISAN-HERRAZ M ; MARTINEZ-MARTINEZ S et al.** A Novel Systems-Biology Algorithm for the Analysis of Coordinated Protein Responses Using Quantitative Proteomics. *Molecular & Cellular Proteomics*, 2016, vol. 15, 1740-60

- **TREVISAN-HERRAZ M ; BAGWAN N ; GARCÍA-MARQUÉS F et al.** SanXoT: a modular and versatile package for the quantitative analysis of high-throughput proteomics experiments. *Bioinformatics,* 2019, vol. 35, 1594-6
- **KUHN M.** Building Predictive Models in R Using the caret Package. *Journal of Statistical Software,* 2008, vol. 28, 1-26
- **TIBSHIRANI R ; BIEN J ; FRIEDMAN J et al.** Strong Rules for Discarding Predictors in Lasso-Type Problems. *Journal of the Royal Statistical Society Series B: Statistical Methodology,* 2012, vol. 74, 245-66
- **WICKHAM H.** ggplot2: Elegant Graphics for Data Analysis. Springer, 2009
- **JAMES G ; WITTEN D ; HASTIE T et al.** An Introduction to Statistical Learning. Springer, 2013